# FASCICULE DE BREVET EUROPEEN

(11) **EP 3 353 165 B1**
(45) Date de publication et mention de la délivrance du brevet: **08.04.2020**
(21) Numéro de dépôt: 16774645.2
(22) Date de dépôt: 23.09.2016
(51) Int. Cl.: C07D 403/04, C07D 205/085, A61K 31/397, A61K 31/4025, A61P 3/00

(54) **1,4-DI-(4-METHYLTHIOPHENYL)-3-PHTALOYLAZETIDINE-2-ONE ET SES DERIVES**
1,4-DI-(4-METHYLTHIOPHENYL)-3-PHTALOYLAZETIDIN-2-ON UND DERIVATE DAVON
1,4-DI-(4-METHYLTHIOPHENYL)-3-PHTALOYLAZETIDINE-2-ONE AND THE DERIVATIVES THEREOF

(30) Priorité: 25.09.2015 FR 1559067
(43) Date de publication de la demande: 01.08.2018
(73) Titulaire: Universite de Nantes, 44000 Nantes (FR); Université de Bourgogne, 21000 Dijon (FR); Institut National de la Santé et de la Recherche Médicale (INSERM), 75013 Paris (FR)
(72) Inventeur: ROBERT, Jean-Michel, 44200 Nantes (FR); TROY-FIORAMONTI, Stéphanie, 33600 PESSAC (FR); DEMIZIEUX, Laurent, 21000 Dijon (FR); DEGRACE, Pascal, 21000 Dijon (FR)
(74) Mandataire: Regimbeau
(86) Numéro de dépôt international: PCT/EP2016/072735
(87) Numéro de publication internationale: WO 2017/050990

(56) Documents cités:
- WO-A1-2007/059871
- WO-A2-2008/039829
- US-A1- 2006 069 080
- HONGWU WANG ET AL: "Identification of Novel Cannabinoid CB1 Receptor Antagonists by Using Virtual Screening with a Pharmacophore Model", JOURNAL OF MEDICINAL CHEMISTRY, vol. 51, no. 8, 1 avril 2008 (2008-04-01), pages 2439-2446, XP055232708, US ISSN: 0022-2623, DOI: 10.1021/jm701519h

## Description

### INTRODUCTION

La présente invention concerne le composé 1,4-di-(4-méthylthiophényl)-3-phtaloylazétidine-2-one et ses dérivés, leur procédé de synthèse ainsi que leurs utilisations, en particulier leur utilisation thérapeutique, notamment dans le traitement de pathologies associées à une hyperactivité du système endocannabinoïde, ainsi que leur utilisation à visée purement esthétique, permettant notamment la perte de poids.

Le système endocannabinoïde (SEC) est un système complexe qui comprend les récepteurs cannabinoïdes, leurs ligands endogènes (i.e. les endocannabinoïdes AEA et 2-AG, plus connus respectivement sous le nom d'anandamide et de 2-arachidonoylglycérol) ainsi que de nombreuses enzymes métaboliques qui catalysent la formation et la dégradation des endocannabinoïdes. Il est désormais bien admis que les endocannabinoïdes sont produits « à la demande » à partir de précurseurs lipidiques membranaires, et que leurs effets biologiques sont relayés plus particulièrement par deux récepteurs à sept domaines transmembranaires couplés aux protéines G, à savoir les récepteurs CB1 et/ou CB2.

Ces derniers sont présents dans de nombreux organes et tissus du corps humain : en effet, outre le cerveau et le système immunitaire, où leur expression est prédominante, les récepteurs CB1 et CB2 ont été identifiés, entre autres, dans l'intestin (Di Carlo et al., 2003), la vessie (Pertwee, 2001), le tissu adipeux (Cota et al., 2003), le foie (Osei-Hyiaman et al., 2005), les testicules (Gye et al., 2001), l'utérus (Das et al., 1995), la rétine (Buckley et al., 1998), l'endothélium vasculaire (Liu et al., 2003), et le muscle.

De par leur vaste localisation tissulaire, ces récepteurs sont impliqués dans la régulation de nombreuses fonctions biologiques mais aussi dans des processus physiopathologiques divers et variés. Une dérégulation du système endocannabinoïde résultant d'une expression altérée des récepteurs endocannabinoïdes CB1 et/ou CB2, des enzymes métabolisant les endocannabinoïdes, et/ou impliquant les voies de synthèse des endocannabinoïdes, a en effet pu être observée dans une myriade de pathologies. Parmi celles-ci, l'obésité et les désordres métaboliques associés, le diabète et ses complications, des maladies hépatiques, rénales et cardiovasculaires, l'ostéoporose, le cancer et les troubles de la fertilité seraient associés à une hyperactivité du système endocannabinoïde, tandis que les maladies inflammatoires de l'intestin et des maladies mentales et neurodégénératives seraient associées à une sous-activité dudit système (Di Marzo, 2008 ; Izzo et al., 2010 ; Pacher et al., 2013 ; Maccarone et al., 2015).

Dans le cas de l'obésité, il a été clairement établi que l'hyperactivation du système endocannabinoïde conduit à une stimulation de l'appétit, et favorise donc la prise de poids, tout en altérant les paramètres métaboliques tels que l'insulinémie, la résistance à l'insuline, la glycémie, la lipidémie, etc. (Ravinet Trillou C.et al., 2003 ; Di Marzo and Matias, 2005; Despres and Lemieux, 2006).

Diverses stratégies thérapeutiques visant à corriger cette dérégulation du système endocannabinoïde, en agissant directement sur les médiateurs de ce système, à savoir les récepteurs CB1 et/ou CB2, ont par conséquent été développées et décrites extensivement dans la littérature scientifique.

Il a été ainsi démontré que l'activation du système endocannabinoïde à l'aide d'agonistes du récepteur CB1 permet d'induire une relaxation transitoire de l'œsophage, et ainsi de traiter le reflux gastro-oesophagien (Beaumont et al., 2009 ; Lehman et al., 2002), et aussi d'améliorer les symptômes associés à des pathologies intestinales telles que le syndrome de l'intestin irritable ou les ulcères gastriques, en agissant notamment sur la motilité gastrointestinale et l'inflammation (Izzo et al., 2001 ; Izzo et al., 1999 ; Massa et al., 2004).

L'utilisation de composés antagonisant l'action du récepteur CB1, tels que le Rimonabant, s'est quant à elle démontrée efficace dans le traitement de l'obésité et du syndrome métabolique, en agissant non seulement de manière centrale sur la prise alimentaire, mais également de manière périphérique sur l'hyperinsulinémie, la résistance à l'insuline, l'hyperglycémie et la dyslipidémie, permettant ainsi de réduire également les risques cardiovasculaires associés (Ravinet Trillou C.et al., 2003 ; Tam et al., 2010 ; Tam et al., 2012).

Des effets bénéfiques d'une action antagoniste dirigée à l'encontre du récepteur CB1 a également pu être observée dans le cadre du diabète et de ses complications (néphropathies, tubulopathies) (Jourdan et al., 2014), et du développement de la fibrose, en particulier la fibrose hépatique et la fibrose rénale (Teixeira-Clerc et al., 2006).

Les agonistes et antagonistes des récepteurs cannabinoïdes, en particulier des récepteurs CB1, présentent donc un intérêt thérapeutique majeur dans les pathologies impliquant une dérégulation du système endocannabinoïde.

Néanmoins, des effets secondaires importants ont été observés suite à l'utilisation prolongée de ces molécules, en particulier lorsque celles-ci franchissent la barrière hémato-encéphalique. Parmi ces molécules, le Rimonabant, initialement destiné à traiter l'obésité, a dû être retiré du marché en 2008 en raison de ses effets psychiatriques dépressogènes consécutifs à son action sur les récepteurs CB1 centraux. Le document WO2008/039829 décrit aussi des composés utiles pour traiter les maladies cardiaques, l'obésité et le diabète.

Il existe donc un besoin pour développer des nouveaux inhibiteurs des récepteurs CB1, qui diffusent peu ou pas du tout dans le système nerveux central, ceci afin de limiter, voire d'annihiler complètement ces effets psychotropes délétères.

La présente invention propose de satisfaire ce besoin à l'aide de composés inédits agissant sur les récepteurs CB1 périphériques.

Les Inventeurs ont en effet découvert de manière surprenante que le composé 1,4-di-(4-méthylthiophényl)-3-phtaloylazétidine-2-one et ses analogues structuraux présentent des caractéristiques physicochimiques qui leur confèrent un profil pharmacocinétique induisant une activité agoniste inverse préférentielle au niveau des récepteurs CB1 périphériques. Les données expérimentales divulguées ci-après démontrent par ailleurs que ces molécules exercent des effets bénéfiques non seulement sur le métabolisme des glucides et des lipides, sur la tolérance au glucose, la sensibilité à l'insuline et la masse corporelle chez la souris obèse, mais également sur la motilité gastro-intestinale, et ceci sans aucune toxicité hépatique. Ces nouveaux composés ouvrent donc la voie à de nouvelles stratégies thérapeutiques non seulement pour les troubles métaboliques associés à l'obésité et les défauts de motricité gastro-intestinale, mais également pour toutes les pathologies associées à une hyperactivité du système endocannabinoïde impliquant préférentiellement les récepteurs CB1.

La présente invention propose par conséquent un agoniste inverse sélectif des récepteurs CB1 périphériques de formule (I) telle que définie ci-après relative à la 1,4-di-(4-méthylthiophényl)-3-phtaloylazétidine-2-one et à ses dérivés, son procédé de synthèse, et ses applications, notamment thérapeutiques et non-thérapeutiques.

### Copie propre

### DESCRIPTION DETAILLEE DE L'INVENTION

La présente description divulgue un composé de formule générale suivante : 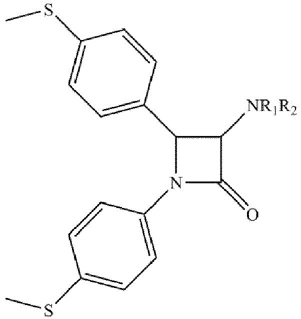 dans laquelle :
- R₁ et R₂, identiques ou différents, représentent un atome d'hydrogène ou un groupe COR₃, SO₂R₄ ou CONR₅R₆; ou forment ensemble avec l'atome d'azote qui les porte un hétérocycle à 5 ou 6 chainons comprenant au moins un hétéroatome supplémentaire, groupe C=O, groupe aryle ou groupe hétéroaryle ;
- R₃, R₄, R₅ et R₆ représentent, indépendamment les uns des autres, un atome d'hydrogène, ou un groupe aryle ou hétéroaryle, ledit groupe étant éventuellement substitué par un ou plusieurs groupe(s) choisi(s) parmi un atome d'halogène, OR₇, NR₈R₉, SR₁₀, S(O)R₁₁, SO₂R₁₂, SO₂NR₁₃R₁₄, OCOR₁₅, NR₁₆COR₁₇, NR₁₈C(O)OR₁₉, CO₂R₂₀, CONR₂₁R₂₂, OCO₂R₂₃, OCONR₂₄R₂₅, COR₂₆, nitro (NO₂), cyano (CN), oxo (=O) et CF₃ ; et
- R₇ à R₂₆ représentent, indépendamment les uns des autres, un atome d'hydrogène ou un groupe (C₁-C₆)alkyle, aryle ou aryl-(C₁-C₆)alkyle,
ou un sel et/ou un solvate pharmaceutiquement acceptable de celui-ci.

La présente invention a ainsi pour premier objet un composé de formule générale (I) suivante : 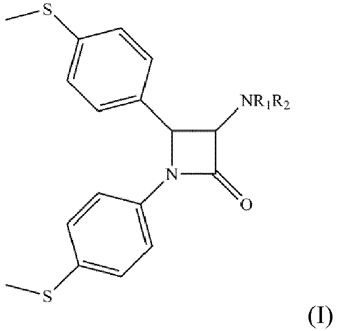 dans laquelle :
- R₁ et R₂, identiques ou différents, représentent un atome d'hydrogène ou un groupe COR₃, SO₂R₄ ou CONR₅R₆; ou forment ensemble avec l'atome d'azote qui les porte un hétérocycle à 5 ou 6 chainons comprenant au moins un hétéroatome supplémentaire, groupe C=O ou groupe aryle;
- R₃, R₄, R₅ et R₆ représentent, indépendamment les uns des autres, un atome d'hydrogène, ou un groupe aryle, ledit groupe étant optionnellement substitué par un ou plusieurs groupe(s) choisi(s) parmi un atome d'halogène, OR₇, NR₈R₉, SR₁₀, S(O)R₁₁, SO₂R₁₂, SO₂NR₁₃R₁₄, OCOR₁₅, NR₁₆COR₁₇, NR₁₈C(O)OR₁₉, CO₂R₂₀, CONR₂₁R₂₂, OCO₂R₂₃, OCONR₂₄R₂₅, COR₂₆, nitro (NO₂), cyano (CN), oxo (=O) et CF₃ ; et
- R₇ à R₂₆ représentent, indépendamment les uns des autres, un atome d'hydrogène ou un groupe (C₁-C₆)alkyle, aryle ou aryl-(C₁-C₆)alkyle,
ou un sel et/ou un solvate pharmaceutiquement acceptable de celui-ci.

Les stéréoisomères des composés de formule générale (I) font également partie de la présente invention, notamment le diastéréoisomère *trans,* ainsi que leurs mélanges.

Par « stéréoisomère », on entend, au sens de la présente invention, un isomère géométrique (ou isomère de configuration) ou un isomère optique.

Les isomères géométriques résultent de la position différente des substituants sur une double liaison ou sur un cycle qui peut avoir alors une configuration Z ou E, encore appelée *cis* ou *trans.*

Les isomères optiques résultent notamment de la position différente dans l'espace des substituants sur un atome de carbone comprenant 4 substituants différents. Cet atome de carbone constitue alors un centre chiral ou asymétrique. Les isomères optiques comprennent les diastéréoisomères et les énantiomères. Les isomères optiques qui sont des images l'un de l'autre dans un miroir mais non superposables sont désignés par « énantiomères ». Les isomères optiques qui ne sont pas des images superposables l'un de l'autre dans un miroir sont désignés par « diastéréoisomères ».

Un mélange contenant des quantités égales de deux formes énantiomères individuelles de chiralité opposée est désigné par « mélange racémique ».

Les tautomères des composés de formule générale (I) font également partie de la présente invention.

Par « tautomère », on entend, au sens de la présente invention, un isomère de constitution du composé obtenu par prototropie, c'est-à-dire par migration d'un atome d'hydrogène et changement de localisation d'une double liaison. Les différents tautomères d'un composé sont généralement interconvertibles et présents en équilibre en solution, dans des proportions qui peuvent varier selon le solvant utilisé, la température ou encore le pH.

Dans la présente invention, on entend désigner par « pharmaceutiquement acceptable » ce qui est utile dans la préparation d'une composition pharmaceutique qui est généralement sûr, non toxique et ni biologiquement ni autrement non souhaitable et qui est acceptable pour une utilisation vétérinaire de même que pharmaceutique humaine.

On entend par « sel pharmaceutiquement acceptable » d'un composé, un sel qui est pharmaceutiquement acceptable, comme défini ici, et qui possède l'activité pharmacologique souhaitée du composé parent.

Les sels pharmaceutiquement acceptables comprennent notamment :
(1) les sels d'addition d'acide pharmaceutiquement acceptable formés avec des acides inorganiques pharmaceutiquement acceptables tels que l'acide chlorhydrique, l'acide bromhydrique, l'acide sulfurique, l'acide nitrique, l'acide phosphorique et similaires ; ou formés avec des acides organiques pharmaceutiquement acceptables tels que l'acide acétique, l'acide benzènesulfonique, l'acide benzoïque, l'acide camphosulfonique, l'acide citrique, l'acide éthane-sulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p-toluènesulfonique, l'acide triméthylacétique, l'acide trifluoroacétique et similaires, et
(2) les sels d'addition de base pharmaceutiquement acceptable formés lorsqu'un proton acide présent dans le composé parent est soit remplacé par un ion métallique, par exemple un ion de métal alcalin, un ion de métal alcalino-terreux ou un ion d'aluminium ; soit coordonné avec une base organique pharmaceutiquement acceptable telle que la diéthanolamine, l'éthanolamine, N-méthylglucamine, la triéthanolamine, la trométhamine et similaires ; ou avec une base inorganique pharmaceutiquement acceptable telle que l'hydroxyde d'aluminium, l'hydroxyde de calcium, l'hydroxyde de potassium, le carbonate de sodium, l'hydroxyde de sodium et similaires.

Il pourra s'agir d'un sel de sodium lorsque le composé comporte une fonction acide.

Ces sels peuvent être synthétisés à partir des composés de l'invention contenant une partie basique ou acide et les acides ou bases correspondants selon les méthodes chimiques conventionnelles.

Les solvates pharmaceutiquement acceptables des composés de la présente invention comprennent les solvates conventionnels tels que ceux formés lors de la dernière étape de préparation des composés de l'invention du fait de la présence de solvants. A titre d'exemple on peut citer les solvates dus à la présence d'eau (hydrates) ou d'éthanol.

Le terme « halogène » représente un fluor, chlore, brome ou iode.

Par groupement « (C₁-C₆)alkyle », on entend, au sens de la présente invention, une chaîne hydrocarbonée saturée, linéaire ou ramifiée, comportant 1 à 6, notamment 1 à 4, atomes de carbone. A titre d'exemple, on peut citer les groupes méthyle, éthyle, propyle, isopropyle, butyle, isobutyle, sec-butyle, tert-butyle, pentyle ou encore hexyle.

Par « aryle », on entend, au sens de la présente invention, un groupement hydrocarboné aromatique, comportant de préférence de 6 à 10 atomes de carbone, et comprenant un ou plusieurs cycles accolés, comme par exemple un groupement phényle ou naphtyle. Avantageusement, il s'agit du phényle.

Par « aryl-(C₁-C₆)alkyle », on entend, au sens de la présente invention, un groupe aryle tel que défini ci-dessus, lié au reste de la molécule par l'intermédiaire d'une chaîne (C₁-C₆)alkyle telle que définie ci-dessus. A titre d'exemple, on peut citer le groupe benzyle.

Par « hétéroaryle », on entend, au sens de la présente invention, un groupe aryle tel que défini ci-dessus, dans lequel 1 à 4, notamment 1 ou 2, atomes de carbone sont chacun remplacés, indépendamment les uns des autres, par un hétéroatome choisi parmi N, O et S.

Selon un mode de réalisation particulier de l'invention, R₁ et R₂ forment ensemble avec l'atome d'azote qui les porte un hétérocycle à 5 ou 6 chainons comprenant au moins un, de préférence un ou deux, hétéroatome supplémentaire, notamment un ou deux atome(s) d'azote (N), groupe C=O, groupe aryle, notamment phényle, ou groupe hétéroaryle, notamment pyridine.

De manière préférée, R₁ et R₂ forment ensemble avec l'atome d'azote qui les porte un hétérocycle de formule (II) ou (III) suivante : 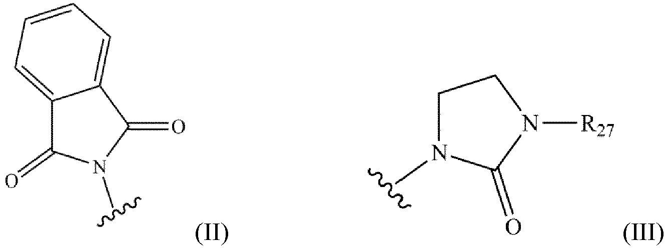 dans laquelle R₂₇ représente un atome d'hydrogène ou un groupe COR₃ ou SO₂R₄, R₃ et R₄ étant tels que définis dans la revendication 1, en particulier R₂₇ représente un atome d'hydrogène.

Selon un autre mode de réalisation particulier de l'invention :
- R₁ et R₂, identiques ou différents, représentent un atome d'hydrogène ou un groupe COR₃, SO₂R₄ ou CONR₅R₆ ; et
- R₃, R₄, R₅ et R₆ représentent, indépendamment les uns des autres, un atome d'hydrogène, ou un groupe aryle, de préférence un phényle, ou hétéroaryle, tel qu'une pyridine, ledit groupe étant optionnellement substitué par un ou plusieurs groupe(s) choisi(s) parmi un atome d'halogène, OR₇, NR₈R₉, SR₁₀, S(O)R₁₁, SO₂R₁₂, SO₂NR₁₃R₁₄, OCOR₁₅, NR₁₆COR₁₇, NR₁₈C(O)OR₁₉, CO₂R₂₀, CONR₂₁R₂₂, OCO₂R₂₃, OCONR₂₄R₂₅, COR₂₆, nitro (NO₂), cyano (CN) et CF₃, avantageusement un atome d'halogène, OR₇, NR₈R₉, SR₁₀, S(O)R₁₁, SO₂R₁₂, OCOR₁₅, CO₂R₂₀, OCO₂R₂₃, COR₂₆, nitro (NO₂), cyano (CN) et CF₃, avantageusement un atome d'halogène, OR₇, NR₈R₉, SO₂R₁₂, et CF₃ ; R₇ à R₂₆ étant tels que définis ci-dessus.

De manière préférée :
- R₁ et R₂, identiques ou différents, représentent un atome d'hydrogène ou un groupe COR₃, SO₂R₄ ou CONR₅R₆ ;
- R₃, R₄ et R₅ représentent, indépendamment les uns des autres, un groupe aryle, de préférence un phényle, optionnellement substitué par un groupe choisi parmi un atome d'halogène, OR₇, NR₈R₉, SR₁₀, S(O)R₁₁, SO₂R₁₂, SO₂NR₁₃R₁₄, OCOR₁₅, NR₁₆COR₁₇, NR₁₈C(O)OR₁₉, CO₂R₂₀, CONR₂₁R₂₂, OCO₂R₂₃, OCONR₂₄R₂₅, COR₂₆, nitro (NO₂), cyano (CN) et CF₃, avantageusement un atome d'halogène, OR₇, NR₈R₉, SR₁₀, S(O)R₁₁, SO₂R₁₂, OCOR₁₅, CO₂R₂₀, OCO₂R₂₃, COR₂₆, nitro (NO₂), cyano (CN) et CF₃, avantageusement un atome d'halogène, OR₇, NR₈R₉, SO₂R₁₂, et CF₃, R₇ à R₂₆ étant tels que définis ci-dessus ; et
- R₆ représente un atome d'hydrogène.

De préférence, R₁ est un atome d'hydrogène et R₂ représente un groupe COR₃, SO₂R₄ ou CONR₅R₆, avec R₃, R₄, R₅ et R₆ étant tels que définis ci-dessus.

Selon un autre mode de réalisation préféré de l'invention, R₁ est un atome d'hydrogène et R₂ représente un groupe COR₃, R₃ étant un aryle, de préférence un phényle, optionnellement substitué par un groupe choisi parmi un atome d'halogène, OR₇, NR₈R₉, SR₁₀, S(O)R₁₁, SO₂R₁₂, SO₂NR₁₃R₁₄, OCOR₁₅, NR₁₆COR₁₇, NR₁₈C(O)OR₁₉, CO₂R₂₀, CONR₂₁R₂₂, OCO₂R₂₃, OCONR₂₄R₂₅, COR₂₆, nitro (NO₂), cyano (CN) et CF₃, avantageusement un atome d'halogène, OR₇, NR₈R₉, SR₁₀, S(O)R₁₁, SO₂R₁₂, OCOR₁₅, CO₂R₂₀, OCO₂R₂₃, COR₂₆, nitro (NO₂), cyano (CN) et CF₃, avantageusement un atome d'halogène, OR₇, NR₈R₉, SO₂R₁₂, et CF₃, avantageusement un atome d'halogène, SO₂CH₃, et CF₃, R₇ à R₂₆ étant tels que définis ci-dessus.

Selon un autre mode de réalisation particulier de l'invention, R₁ est un atome d'hydrogène et R₂ représente un groupe SO₂R₄, R₄ étant un aryle, de préférence un phényle, optionnellement substitué par un groupe choisi parmi un atome d'halogène, OR₇, NR₈R₉, SR₁₀, S(O)R₁₁, SO₂R₁₂, SO₂NR₁₃R₁₄, OCOR₁₅, NR₁₆COR₁₇, NR₁₈C(O)OR₁₉, CO₂R₂₀, CONR₂₁R₂₂, OCO₂R₂₃, OCONR₂₄R₂₅, COR₂₆, nitro (NO₂), cyano (CN) et CF₃, avantageusement un atome d'halogène, OR₇, NR₈R₉, SR₁₀, S(O)R₁₁, SO₂R₁₂, OCOR₁₅, CO₂R₂₀, OCO₂R₂₃, COR₂₆, nitro (NO₂), cyano (CN) et CF₃, avantageusement un atome d'halogène, OR₇, NR₈R₉, SO₂R₁₂, et CF₃, avantageusement un atome d'halogène, SO₂CH₃, et CF₃, R₇ à R₂₆ étant tels que définis ci-dessus.

Selon un autre mode de réalisation particulier de l'invention, R₁ est un atome d'hydrogène et R₂ représente un groupe CONR₅R₆, R₆ étant un atome d'hydrogène et R₅ étant un aryle, de préférence un phényle, optionnellement substitué par un groupe choisi parmi un atome d'halogène, OR₇, NR₈R₉, SR₁₀, S(O)R₁₁, SO₂R₁₂, SO₂NR₁₃R₁₄, OCOR₁₅, NR₁₆COR₁₇, NR₁₈C(O)OR₁₉, CO₂R₂₀, CONR₂₁R₂₂, OCO₂R₂₃, OCONR₂₄R₂₅, COR₂₆, nitro (NO₂), cyano (CN) et CF₃, avantageusement un atome d'halogène, OR₇, NR₈R₉, SR₁₀, S(O)R₁₁, SO₂R₁₂, OCOR₁₅, CO₂R₂₀, OCO₂R₂₃, COR₂₆, nitro (NO₂), cyano (CN) et CF₃, avantageusement un atome d'halogène, OR₇, NR₈R₉, SO₂R₁₂, et CF₃, avantageusement un atome d'halogène, SO₂CH₃, et CF₃, R₇ à R₂₆ étant tels que définis ci-dessus.

Les composés de la présente invention pourront être plus préférablement choisis parmi les composés IA à IF décrits ci-après, et leurs sels et/ou solvates pharmaceutiquement acceptables :

| | |
|---|---|
| Composé (IA) - JM-00.266 | Composé (IB) |
| 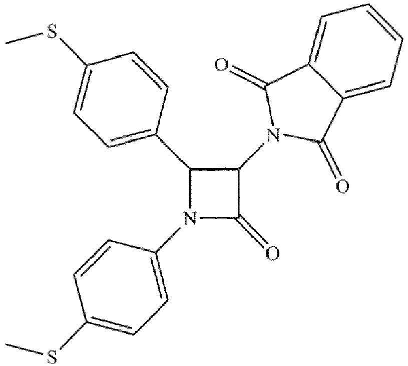 | 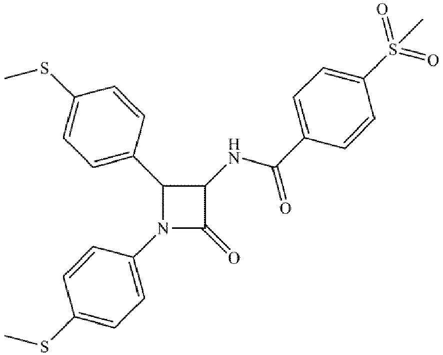 |
| Composé (IC) | Composé (ID) |
| 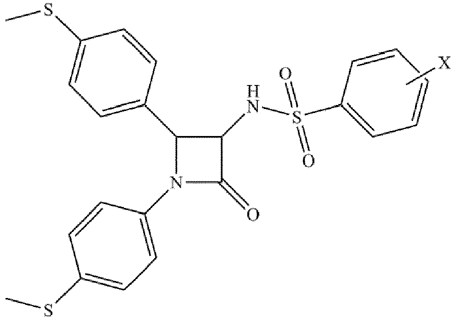 | 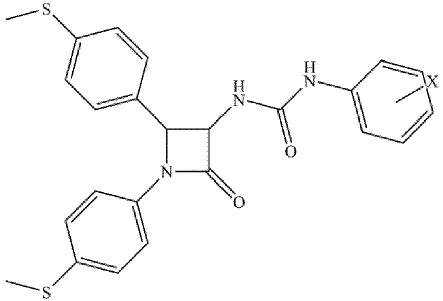 |
| Composé (IE) | Composé (IF) |
| 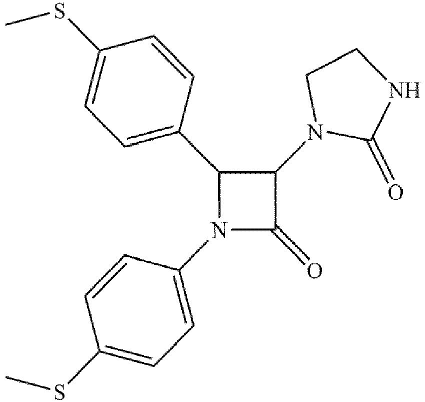 | 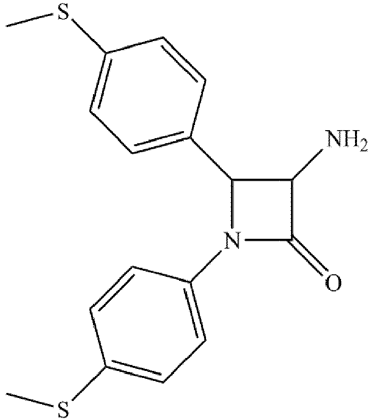 |

dans lesquels X représente un atome d'hydrogène, un halogène ou CF₃.

Selon un mode de réalisation plus particulièrement préféré de l'invention, le composé de formule (I) selon l'invention est le composé de formule (IA) : 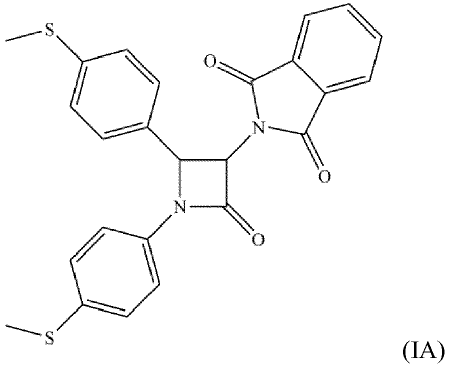 ou un sel et/ou un solvate pharmaceutiquement acceptable de celui-ci.

La présente description a également pour objet des procédés de préparation des composés de formule (I) selon l'invention.

Un procédé de préparation d'un composé de formule (I) selon l'invention comprend les étapes suivantes :
(i) condensation du 4-méthylthiobenzaldéhyde avec la 4-méthylthioaniline pour obtenir le composé de formule (IV) suivante : 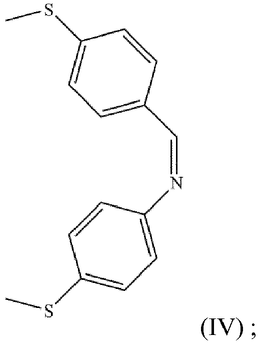
(ii) cycloaddition de Staudinger entre le composé de formule (IV) obtenu et un cétène de formule (V) suivante : 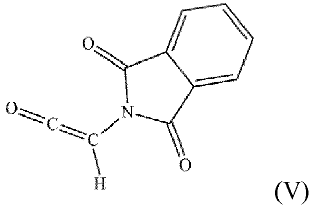 pour obtenir le composé de formule (IA) suivante : 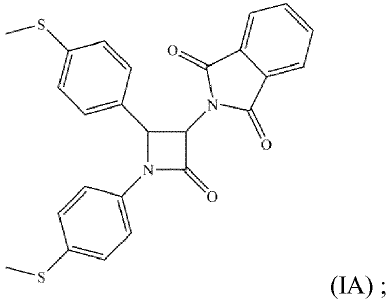
(iii) optionnellement, déprotection de la fonction amine phtaloylée du composé de formule (IA), de préférence par action de la méthylhydrazine, pour obtenir le composé de formule (IF) suivante : 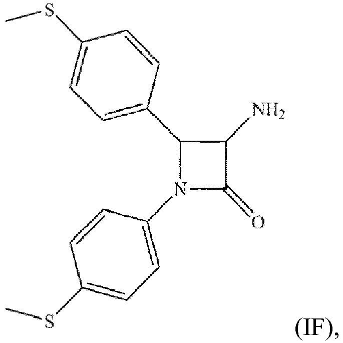 puis, optionnellement, couplage du composé de formule (IF) ainsi obtenu avec un composé de formule R₁-X et/ou R₂-X', dans laquelle R₁-X et R₂-X' sont des formes activées, telles que chlorures d'acyles, chlorures de sulfonyles et isocyanates d'aryles, des groupements R₁ et R₂ tels que définis ci-dessus ; et
(iv) récupération du composé obtenu à l'étape (ii) ou à l'étape (iii).

A titre d'exemple de chlorures d'acyles dans l'étape (ii), on peut utiliser selon un mode particulier le chlorure de phtaloylglycinyle.

Selon un mode de réalisation préféré, le procédé de l'invention vise à préparer un composé de formule (IA) : 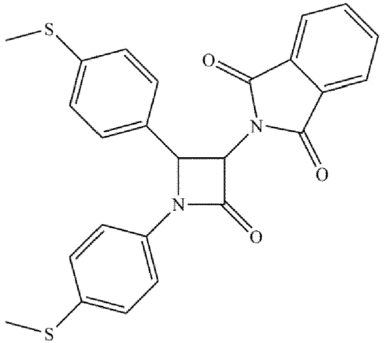 correspondant à un composé de formule (I) dans laquelle R₁ et R₂ forment ensemble avec l'atome d'azote qui les porte un hétérocycle de formule (II).

Ce procédé comprend les étapes suivantes :
(i) condensation du 4-méthylthiobenzaldéhyde avec la 4-méthylthioaniline pour obtenir le composé de formule (IV) suivante : 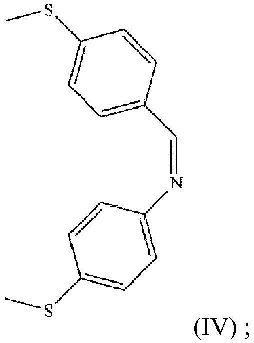
(ii) cycloaddition de Staudinger entre le composé de formule (IV) obtenu à l'étape (i) et un cétène de formule (V) suivante : 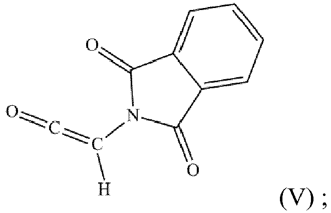 puis récupération du composé de formule (IA).

La réaction de condensation (déshydratation intermoléculaire) sera réalisée avantageusement en présence d'un solvant, tel que le toluène et d'un agent de fixation de l'eau produite au cours de la réaction, tel que le sulfate de sodium anhydre.

La cycloaddition de Staudinger, aussi appelée cycloaddition [2+2] cétène-imine, est une réaction chimique connue de l'homme du métier. Cette réaction peut être mise en œuvre dans du dichloroéthane (DCE), à température ambiante et sous atmosphère inerte telle que sous azote ou sous argon, préférablement sous azote.

Le cétène (V) pourra avantageusement être généré *in-situ* par action d'une base telle que la triéthylamine (TEA) sur le chlorure d'acide de la phtaloylglycine. Ce chlorure d'acide pourra être obtenu par des méthodes connues de l'homme du métier, notamment soit de façon externe par action du chlorure de thionyle sur la *N-*phtaloylglycine commerciale soit directement dans le milieu réactionnel au moyen d'un agent de couplage, tel que le dichlorophosphate de phényle en présence d'un accepteur de protons tel que la triéthylamine.

Un tel procédé est illustré plus en détails sur le schéma 1 suivant.

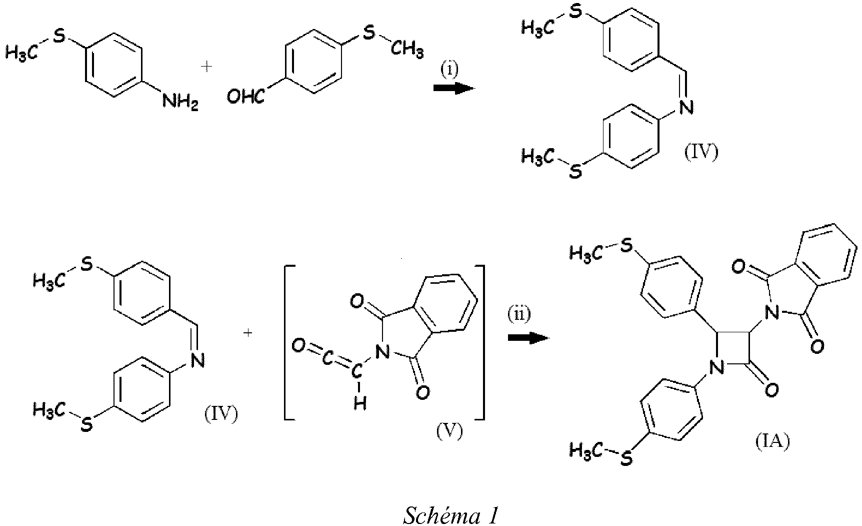

Ce procédé permet ainsi d'obtenir le composé (IA) qui pourra servir comme réactif de départ dans le procédé de préparation des autres composés de formule (I).

La présente invention concerne également un procédé de préparation des composés de formule (I), utilisant comme produit de départ le composé de formule (IA) et comprenant les étapes suivantes :
(i) condensation du 4-méthylthiobenzaldéhyde avec la 4-méthylthioaniline pour obtenir le composé de formule (IV) ;
(ii) cycloaddition de Staudinger entre le composé de formule (IV) obtenu à l'étape (i) et un cétène de formule (V) ;
(iii) déprotection de la fonction amine phtaloylée du composé de formule (IA) obtenu à l'étape (ii), de préférence par action de la méthylhydrazine, pour obtenir le composé de formule (IF) suivante : 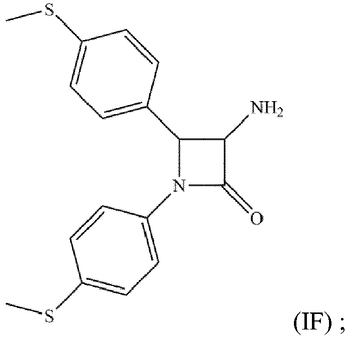 puis, optionnellement, couplage du composé de formule (IF) ainsi obtenu avec un composé de formule R₁-X et/ou R₂-X', dans laquelle R₁-X et R₂-X' sont des formes activées, telles que des chlorures d'acyles, chlorures de sulfonyles et isocyanates d'aryles, des groupements R₁ et R₂ tels que définis ci-dessus ; et
(iv) récupération du composé obtenu à l'étape (iii).

A titre d'exemple de chlorures d'acyles dans l'étape (ii), on peut utiliser selon un mode particulier le chlorure de phtaloylglycinyle.

Selon un mode particulier, le procédé de préparation des composés de formule (I), utilisant comme produit de départ le composé de formule (IA) peut comprendre les étapes suivantes :
(i) condensation du 4-méthylthiobenzaldéhyde avec la 4-méthylthioaniline pour obtenir le composé de formule (IV)
(ii) cycloaddition de Staudinger entre le composé de formule (IV) obtenu à l'étape (i) et un cétène de formule (V) obtenu *in situ,* au moyen d'un agent de couplage, tel que le dichlorophosphate de phényle en présence d'un accepteur de protons tel que la triéthylamine ;
(iii) déprotection de la fonction amine phtaloylée du composé de formule (IA) obtenu à l'étape (ii),, de préférence par action de la méthylhydrazine, pour obtenir le composé de formule (IF), puis, optionnellement, couplage, au moyen d'un agent de couplage, tel que le dichlorophosphate de phényle en présence d'un accepteur de protons tel que la triéthylamine, du composé de formule (IF) ainsi obtenu avec une N-phtaloylglycine pour obtenir un dérivé de formule 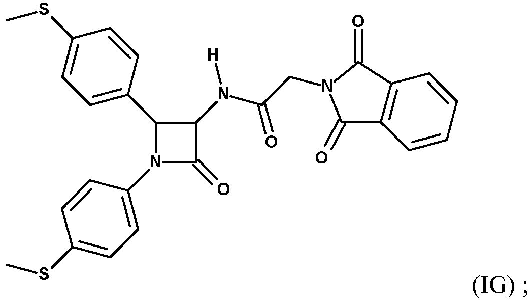 et
(iv) récupération du composé obtenu à l'étape (ii) ou à l'étape (iii).

Ainsi, un autre objet de l'invention concerne un dérivé / analogue du composé (IA) de formule (IG) : 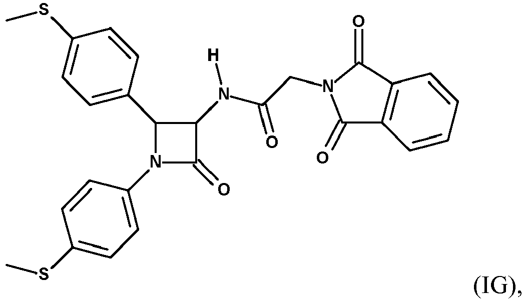 ou un sel et/ou un solvate pharmaceutiquement acceptable de celui-ci.

La réaction de déprotection de la fonction amine phtaloylée du composée (IA) est une réaction typique, bien connue de l'homme du métier. Cette déprotection sera réalisée avantageusement par action de l'hydrate d'hydrazine ou de la méthylhydrazine, de préférence de la méthylhydrazine, sur le composé (IA), de préférence dans le dichlorométhane. Cette réaction permet d'obtenir le composé (IF) correspondant à un composé de formule (I) dans laquelle R₁ et R₂ représente chacun un atome d'hydrogène.

Selon un mode de réalisation particulier, le composé (IF) est récupéré directement, sans que la réaction de couplage ait lieu.

Selon un autre mode de réalisation particulier, le composé (IF) est utilisé dans la suite du procédé comme intermédiaire de synthèse pour préparer les composés de formule (I) autres que le composé (IA).

La présente invention a donc également pour objet le composé (IF) en tant qu'intermédiaire de synthèse des composés de formule (I) autres que le composé (IA).

La réaction de couplage pourra être réalisée dans des conditions expérimentales bien connues de l'homme du métier, avantageusement à température ambiante en présence d'un solvant, tel que le dichloroéthane, et d'une base, telle que la triéthylamine (TEA).

Par « forme activée » d'un groupement chimique, on entend, au sens de la présente invention, ledit groupement chimique modifié de manière à le rendre plus actif vis-à-vis des nucléophiles. Ces formes activées sont bien connues de l'homme du métier et peuvent être en particulier un chlorure d'acyle, un chlorure de sulfonyle ou un isocyanate d'aryle. Comme exemple de chlorure d'acyle, on peut en particulier utiliser le chlorure de phtaloylglycinyle.

Les procédés décrits ci-dessus pourront être complétés, le cas échéant, par toutes manipulations standards décrites dans la littérature, connues de l'homme de métier ou bien encore exemplifiées dans la partie expérimentale, notamment par des réactions de fonctionnalisation, de cyclisation et/ou de protection/déprotection additionnelles.

Une étape ou des étapes additionnelles de salification et/ou de solvatation pourront être réalisées à la fin de ces deux procédés afin d'obtenir un sel et/ou solvate pharmaceutiquement acceptable du composé de formule (I).

L'étape de salification pourra être réalisée dans des conditions bien connues de l'homme du métier, en présence d'un acide ou d'une base pharmaceutiquement acceptable.

Lorsque le composé de formule (I) se trouve sous une forme solvatée, cette solvatation a généralement lieu dans la dernière étape du procédé, le solvant de la forme solvatée étant dans ce cas le solvant du milieu réactionnel.

Le composé de formule (I) obtenu par l'un de ces deux procédés mentionnés ci-dessus pourra être séparé du milieu réactionnel par des méthodes bien connues de l'homme du métier, comme par exemple par extraction, évaporation du solvant ou encore par précipitation et filtration.

Le composé de formule (I) pourra être par ailleurs purifié si nécessaire par des techniques bien connues de l'homme du métier, comme par recristallisation si le composé est cristallin, par distillation, par chromatographie sur colonne sur gel de silice ou encore par chromatographie liquide haute performance (HPLC).

Les Inventeurs ont mis en évidence l'activité agoniste inverse des composés de l'invention à l'encontre des récepteurs endocannabinoïdes CB1 périphériques, et leur intérêt dans le traitement ou la prévention de pathologies associées à une hyperactivité du système endocannabinoïde.

Ainsi, un autre objet de l'invention concerne l'utilisation *in vitro* d'au moins un composé de formule (I) tel que défini ci-dessus en tant qu'agoniste inverse des récepteurs endocannabinoïdes CB1 périphériques, préférablement en tant qu'agoniste inverse sélectif desdits récepteurs. En d'autres termes, l'invention concerne une utilisation d'au moins un composé de formule (I) pour réduire ou inhiber l'activité des récepteurs endocannabinoïdes CB1 périphériques, préférablement de manière sélective.

Par « agoniste inverse », on entend ici un composé qui interagit avec le même récepteur qu'un agoniste naturel de ce récepteur mais produit l'effet pharmacologique opposé, et réduit, voire inhibe, l'activité dudit récepteur, en particulier son activité basale. Un agoniste inverse est capable de se lier sur un site de fixation différent de l'agoniste naturel entraînant un changement de conformation du récepteur et empêchant ainsi la fixation de l'agoniste naturel. Dans le contexte de la présente invention, les agonistes naturels sont les endocannabinoïdes.

Par « agoniste inverse sélectif », on entend ici un agoniste inverse tel que défini ci-dessus, qui se fixe préférentiellement sur un type unique de récepteur, sans affecter ou de manière minime d'autres récepteurs, en particulier les récepteurs apparentés. Dans le contexte de la présente invention, le composé de formule (I) selon l'invention est un agoniste inverse agissant sélectivement sur les récepteurs endocannabinoïdes CB1, i.e. se liant préférentiellement auxdits récepteurs, mais ne se fixant pas ou très faiblement sur les récepteurs endocannabinoïdes CB2.

Par «récepteurs endocannabinoïdes CB1 » ou «récepteurs CB1 », on entend ici les récepteurs à sept domaines transmembranaires couplés aux protéines G, notamment couplés à Gi/0 sensible à la toxine pertussique et aux protéines Gq et Gs, capables d'interagir avec les cannabinoïdes endogènes et exogènes et ainsi d'agir principalement sur au moins une des trois voies de signalisation intracellulaires que constituent l'adénylate cyclase, la voie des protéines kinases activées par des agents mitogènes (MAPK) et certains canaux ioniques. L'activité des récepteurs CB1 peut ainsi être évaluée *in vitro* en mesurant par exemple les concentrations en AMPc suite à la mise en contact de cellules avec un agoniste connu desdits récepteurs, tel que l'anandamide (AEA), ces concentrations diminuant après liaison de cet endocannabinoïde aux récepteurs ; un tel test est décrit dans les exemples décrits ci-après. Chez l'homme, le récepteur endocannabinoïde CB1 est codé par le gène *Cnr1,* qui est localisé sur le chromosome 6 en 6q14-q15 (Hoehe et al., 1991), et se présente sous deux isoformes : CB1, qui est l'isoforme majoritaire de 472 acides aminés et physiologiquement active, et CB1A, qui est une isoforme plus courte (411 acides aminés) et dont l'expression est nettement plus faible que celle de CB1 (de 1/10 à 1/100 selon le site d'expression tissulaire) (Rinaldi-Carmona et al., 1996). A ce jour, le récepteur CB1 a été également identifié chez le rat, la souris, le chat, les oiseaux, les amphibiens et les poissons, et sa séquence protéique est hautement conservée chez les vertébrés.

Le terme « récepteurs endocannabinoïdes CB1 périphériques » se réfère aux récepteurs CB1 tels que définis ci-dessus, qui ne sont pas localisés dans le cerveau, i.e. centralement. Ce terme est par conséquent utilisé ici par opposition aux récepteurs CB1 dits centraux, et inclut les récepteurs CB1 exprimés, sans limitation, dans le tissu adipeux, le foie, les reins, le système gastro-intestinal, la vessie, le muscle squelettique, les tissus cardiovasculaires, les testicules, l'utérus, le système immunitaire, le pancréas, les cellules de la rétine, les cellules endothéliales, les glandes surrénales, les poumons, etc.

L'invention a également pour objet une composition pharmaceutique comprenant en tant que principe actif au moins un composé de formule (I) selon l'invention, et au moins un excipient pharmaceutiquement acceptable.

Par « excipient pharmaceutiquement acceptable », on entend ici un composé de qualité pharmaceutique qui améliore la délivrance, la stabilité ou la biodisponibilité d'un agent actif, et qui peut être métabolisé et est non toxique pour un sujet auquel elle est administrée. Des excipients préférés selon l'invention comprennent l'un quelconque des excipients communément utilisés dans les produits pharmaceutiques, tels que la cellulose microcristalline, le lactose, l'amidon, et la poudre de soja.

Ledit composé de formule (I) est préférablement présent dans la composition selon l'invention dans une quantité suffisante pour inhiber l'activité des récepteurs CB1 périphériques, plus particulièrement dans le cadre d'un traitement prophylactique ou thérapeutique d'une pathologie associée à une hyperactivité du système endocannabinoïde. Les pathologies plus particulièrement concernées sont décrites ci-après.

De préférence, la composition de l'invention comprend de 0,01% à 10%, de préférence de 0,02% à 5%, de manière encore préférée de 0,05 à 1% en poids de la composition d'un ou plusieurs composés de formule (I) selon l'invention.

La composition selon l'invention peut se présenter sous toutes les formes galéniques acceptables dans le contexte de la présente invention. Par exemple, la composition peut être dans une forme adaptée à l'administration orale, sublinguale, sous-cutanée, intramusculaire, intraveineuse, topique, locale, intratrachéale, intranasale, transdermique ou rectale. La forme la plus préférée de la composition pharmaceutique est la voie orale et solide, de préférence sous la forme de gélules ou de comprimés.

La composition décrite peut comprendre en outre un ou plusieurs agents thérapeutiques, par exemple destinés à prévenir ou à traiter une pathologie associée à une hyperactivité du système endocannabinoïde. L'homme du métier peut aisément déterminer l'agent thérapeutique susceptible d'être associé au composé de formule (I) de l'invention, en fonction de la pathologie à prévenir ou à traiter. A titre d'illustration, lorsque la pathologie à prévenir ou à traiter est le diabète et ses complications, et/ou les troubles métaboliques associés à l'obésité, ledit agent peut être choisi parmi un hypolipémiant, un hypocholestérolémiant, un antidiabétique, et/ou un agent anti-obésité. Les hypolipémiants et hypocholestérolémiants selon l'invention incluent, sans limitation, les fibrates tels que alufibrate, béclobrate, bézafibrate, ciprofibrate, clinofibrate, clofibrate, étofibrate, fénofibrate; les statines (inhibiteurs de HMG-CoA reductase), telles que atorvastatine, fluvastatine sodium, lovastatine, pravastatine, rosuvastatine, simvastatine, ou un composé tel que acipimox, aluminum nicotinate, azacostérol, cholestyramine, dextrothyroxine, méglutol, nicéritrol, nicoclonate, acide nicotinique, béta-sitosterin, et tiadénol. Les antidiabétiques selon l'invention incluent, sans limitation, les sulfonylurées, les biguanidines, les inhibiteurs d'alpha-glucosidase, les thiazolidinediones, les métiglinides tel que acarbose, acétohexamide, carbutamide, chlorpropamide, glibenclamide, glibornuride, gliclazide, glimépiride, glipizide, gliquidone, glisoxepide, glybuzole, glymidine, métahexamide, metformine, miglitol, natéglinide, pioglitazone, répaglinide, rosiglitazone, tolazamide, tolbutamide, et voglibose.

L'agent thérapeutique tel que décrit ci-dessus peut également être administré en association avec le composé de formule (I) selon l'invention de manière simultanée, séparée, ou étalée dans le temps. Lorsque le composé selon l'invention et l'agent thérapeutique sont administrés de manière séparée ou étalée dans le temps, ceux-ci pouvant alors être administrés dans des formes pharmaceutiquement distinctes.

Ainsi, un autre objet de l'invention concerne un composé de formule (I) selon l'invention et un agent thérapeutique destinés à prévenir ou à traiter une pathologie associée à une hyperactivité du système endocannabinoïde, en tant que préparation combinée pour une administration simultanée, séparée, ou étalée dans le temps. En d'autres termes, l'invention concerne une utilisation combinée du composé de formule (I) selon l'invention et d'un agent thérapeutique tel que décrit ci-dessus pour une administration simultanée, séparée, ou étalée dans le temps.

L'invention a également pour objet le composé de formule (I) ou la composition pharmaceutique tel(le) que défini(e) ci-dessus, pour son utilisation comme médicament. En d'autres termes, l'invention concerne l'utilisation dudit composé de formule (I) ou de ladite composition pharmaceutique en tant que médicament.

L'invention concerne plus particulièrement le composé de formule (I) ou la composition pharmaceutique tel(le) que défini(e) ci-dessus pour son utilisation dans la prévention ou le traitement de pathologies associées à une hyperactivité du système endocannabinoïde. Autrement dit, l'invention concerne l'utilisation dudit composé de formule (I) ou de ladite composition pharmaceutique pour la fabrication d'un médicament destiné à prévenir ou à traiter des pathologies associées à une hyperactivité du système endocannabinoïde. Plus précisément, l'invention concerne une méthode de prévention ou de traitement de pathologies associées à une hyperactivité du système endocannabinoïde, comprenant une étape d'administration d'une quantité efficace du composé de formule (I) ou de la composition selon l'invention chez un sujet en ayant besoin.

Tels qu'utilisés ici, les termes « prévention » (ou « prévenir ») et « traitement » (ou « traiter ») signifient de manière générale l'obtention d'un effet physiologique ou pharmacologique souhaité en fonction du degré de sévérité des symptômes ou de la maladie d'intérêt. L'effet peut être prophylactique en termes de prévention partielle ou complète des symptômes ou maladie (« prévention »), ou thérapeutique en termes de guérison partielle ou complète des symptômes ou maladie (« traitement »). Le terme « prévention » inclut la capacité à éviter ou à retarder l'apparition ou le développement de symptômes ou d'une maladie avant son apparition. Le terme « traitement» comprend quant à lui l'inhibition des symptômes ou de la maladie (c.-à-d. l'arrêt de son développement), et le soulagement des symptômes ou de la maladie (c.-à-d. régression conduisant à une amélioration). Dans le contexte de la présente invention, la prévention vise à éviter ou à retarder l'apparition d'une hyperactivité du système endocannabinoïde, tandis que le traitement conduit à arrêter et/ou à faire régresser ladite hyperactivité. Il est entendu que ledit traitement ou ladite prévention concerne ici préférablement les sujets pourvus d'un système cannabinoïde endogène, à savoir l'homme et l'animal.

La quantité efficace de composé de formule (I) à administrer à un sujet affecté par une hyperactivité du système endocannabinoïde peut être aisément déterminée par l'homme du métier. Typiquement, une quantité thérapeutiquement efficace d'un agent actif est comprise entre environ 10 mg par jour à environ 1000 mg par jour, préférablement de 10 mg à 100 mg par jour.

Par « hyperactivité du système endocannabinoïde », on entend ici une dérégulation du système endocannabinoïde qui se traduit chez le sujet affecté par une surexpression et/ou suractivité des récepteurs endocannabinoïdes CB1 et/ou CB2, et/ou par une surexpression et/ou suractivité des enzymes métabolisant les endocannabinoïdes, et/ou par un taux anormalement élevé des endocannabinoïdes (e.g. 2AG et/ou anandamide) suite à un dérèglement de leur voie de synthèse. Dans le cadre de la présente invention, ladite hyperactivité est préférablement médiée par les récepteurs CB1, et implique plus particulièrement les récepteurs CB1 périphériques (en association ou non avec les récepteurs CB2). Cette hyperactivité est donc préférentiellement présente dans les tissus dans lesquels sont exprimés les récepteurs CB1 périphériques. L'homme du métier est à même d'identifier les pathologies associées à une hyperactivité du système endocannabinoïde, préférablement médiée par les récepteurs CB1.

Les dites pathologies sont sélectionnées parmi l'obésité et les troubles métaboliques associés à l'obésité (Di Marzo et al., 2005 ; Blüher et al., 2006 ; Côté et al., 2007 ; la résistance à l'insuline (Song et al., 2011; Eckardt et al., 2009), préférablement les troubles métaboliques associés à l'obésité ; le diabète, préférablement de type II, et les complications associées (Matias et al., 2006; Jensen, 2006) ; la stéatose hépatique, alcoolique ou non alcoolique (Osei-Hyiamann et al., 2005; Osei-Hyiaman et al., 2008; Jeong et al., 2008) ; la fibrose hépatique (Teixeira-Clerc et al., 2006) ; la cirrhose; la fibrose rénale (Lecru et al., 2015) ; la néphropathie (Jourdan et al., 2012) ; les cardiomyopathies (Montecucco et Di Marzo, 2012; Rajesh et al., 2012; Slavic et al., 2013; Schaich et al., 2014; Pacher et Kunos, 2013) ; la gastroparésie (Izzo et Sharkey, 2010) ; la perte d'os et/ou de cartilage liée par exemples à l'ostéoporose ou à la périodontite (Tam et al., 2008) ; la perte de muscle, qui est par exemple consécutive à un traumatisme, ou d'origine naturelle (liée à la vieillesse) ou génétique telle que les dystrophies musculaires (e.g. la myopathie de Duchenne, etc) (Iannotti et al., 2014) ; et les troubles de la fertilité liés par exemple à une faible motilité et/ou viabilité des spermatozoïdes ou à un défaut de la nidation de l'ovocyte (Amoako et al., 2014).

De manière encore préférée, les dites pathologies sont sélectionnées parmi l'obésité et les troubles métaboliques qui sont associés à l'obésité, et la gastroparésie. Plus préférablement, les dites pathologies sont sélectionnées parmi les troubles métaboliques associés à l'obésité, et la gastroparésie.

Parmi les troubles métaboliques associés à l'obésité, on peut citer, sans limitation, la résistance à l'insuline, l'intolérance au glucose, les dyslipidémies telles que l'hypertriglycéridémie et l'hypercholestérolémie, le prédiabète et la stéatose hépatique.

Parmi les complications associées au diabète, on peut citer, sans limitation, les maladies oculaires telles que les rétinopathies diabétiques, les oedèmes oculaires et le glaucome, pouvant conduire à la perte de la vue ; les maladies rénales telles que l'insuffisance rénale, la néphropathie diabétique et la glomérulopathie diabétique ; les angiopathies telles que les micro- et macro-angiopathies, les coronopathies et arthériopathies périphériques ; la stéatose hépatique ; les maladies cardiovasculaires ; la dysfonction érectile ; la gastroparésie diabétique ; les maladies neurologiques telles que les neuropathies diabétiques, les neuropathies périphériques et les neuropathies cardiaques autonomes.

De manière préférée, le traitement de l'obésité et/ou des troubles métaboliques de l'obésité pourra être associé à un agent anti-obésité, à un agent hypolipémiant, à un agent hypocholestérolémiant, voire à un antidiabétique, tels que décrits ci-dessus, et/ou à un régime alimentaire permettant de réduire la prise de calories en particulier un régime alimentaire normocalorique équilibré, et ainsi faciliter la perte de poids concomitamment à l'amélioration de la sensibilité à l'insuline, de la tolérance au glucose et de la lipidémie médiée par le composé de formule (I) selon l'invention.

Par « régime alimentaire normocalorique équilibré », on désigne ici un régime alimentaire dont la composition respecte les proportions de glucides / lipides / protéines recommandées par l'ANSES (respectivement 50-55% / 35-40 % / 10 -30%). L'homme du métier saura ainsi adapter le régime alimentaire normocalorique équilibré à utiliser pour le sujet à traiter en fonction de son sexe, poids, taille, âge, et/ou état de santé

Ainsi, selon un mode de réalisation préféré de l'invention, en particulier pour prévenir et/ou traiter l'obésité et/ou les troubles métaboliques qui y sont associés, le sujet est soumis à un régime alimentaire normocalorique équilibré.

De manière encore préférée, ledit sujet est soumis audit un régime alimentaire normocalorique équilibré, simultanément à l'administration du composé de formule (I) ou de la composition pharmaceutique selon l'invention.

Selon un autre mode de réalisation préféré, l'administration du composé de formule (I) ou de la composition pharmaceutique selon l'invention est effectuée avant et/ou pendant le ou les repas du sujet. Ce mode de réalisation peut être combiné ou non à un régime alimentaire normocalorique équilibré tel que décrit ci-dessus. Par « avant le ou les repas », on entend ici que l'administration du composé de formule (I) ou de la composition pharmaceutique selon l'invention est effectuéeau plus 30 minutes avant le repas, de préférence au plus 15 minutes avant le repas et de préférence encore juste avant le repas.

D'après les données expérimentales présentées ci-dessous, l'homme du métier comprendra aisément que le composé de formule (I) selon l'invention peut également être utilisé à des fins uniquement esthétiques, notamment pour favoriser la perte de poids.

Ainsi, dans un autre aspect, l'invention porte sur une utilisation non thérapeutique du composé de formule (I) de l'invention pour promouvoir et/ou accélérer la perte de poids, ou pour ralentir et/ou atténuer la prise de poids, chez un sujet.

En d'autres termes, l'invention porte sur une méthode non thérapeutique pour promouvoir et/ou accélérer la perte de poids, ou pour ralentir et/ou atténuer la prise de poids chez un sujet, ladite méthode comprenant une étape d'administration d'une quantité efficace du composé de l'invention audit sujet.

L'utilisation visée ici est exempt d'effets thérapeutiques (secondaires), i.e. il s'agit d'une utilisation non pas pour la prévention ou le traitement d'une maladie ou d'un de ses symptômes, mais seulement pour améliorer l'aspect esthétique d'une personne. Ainsi, le sujet sur lequel la présente méthode non thérapeutique est pratiquée est préférablement un sujet ne souffrant pas d'une hyperactivité du système endocannabinoïde, telle que l'obésité, et/ou un sujet en bonne santé (i.e. individu sain). Selon un mode particulier, le sujet peut être en surpoids par rapport à sa taille mais non obèse.

Dans le cadre de cette utilisation, la quantité efficace du composé de formule (I) à administrer au sujet peut être aisément déterminée par l'homme du métier. Typiquement, une quantité efficace est comprise entre 10 mg par jour à environ 1000 mg par jour, préférablement de 10 mg à 100 mg par jour.

Selon un mode de réalisation préféré, ledit sujet est soumis à régime alimentaire normocalorique équilibré.

De manière préférée, ledit sujet est soumis audit régime alimentaire normocalorique équilibré, simultanément à l'administration du composé de formule (I) selon l'invention.

Selon un autre mode de réalisation préféré, l'administration du composé de formule (I) est effectuée avant et/ou pendant le ou les repas du sujet. Ce mode de réalisation peut être combiné ou non à un régime alimentaire normocalorique équilibré tel que décrit ci-dessus.

Le composé de l'invention peut également être utilisé dans une composition destinée à favoriser l'amincissement.

Ainsi, selon un autre aspect, l'invention porte sur une composition non thérapeutique, pour promouvoir et/ou accélérer la perte de poids, ou pour ralentir et/ou atténuer la prise de poids, chez un sujet, ladite composition comprenant au moins un composé selon l'invention et au moins un excipient acceptable.

Plus précisément, l'invention porte sur l'utilisation non thérapeutique de ladite composition, pour promouvoir et/ou accélérer la perte de poids, ou pour ralentir et/ou atténuer la prise de poids, chez un sujet.

En d'autres termes, l'invention porte sur une méthode non thérapeutique pour promouvoir et/ou accélérer la perte de poids, ou pour ralentir et/ou atténuer la prise de poids chez un sujet, ladite méthode comprenant une étape d'administration d'une quantité efficace de ladite composition audit sujet.

Ledit composé de formule (I) est préférablement présent dans cette composition dans une quantité suffisante pour promouvoir et/ou accélérer la perte de poids, ou pour ralentir et/ou atténuer la prise de poids.

De préférence, ladite composition comprend de 0,01% à 10%, de préférence de 0,02% à 5%, de manière encore préférée de 0,05% à 1% en poids de la composition d'un ou plusieurs composés de formule (I) selon l'invention.

Ladite composition peut se présenter sous toutes les formes acceptables pour une utilisation non thérapeutique telle que décrite ici. Par exemple, ladite composition peut être sous une forme adaptée à l'administration orale, sublinguale, topique, locale, etc. La forme la plus préférée de ladite composition est adaptée à l'administration orale.

La composition non thérapeutique selon l'invention peut être sous la forme d'une poudre, d'une capsule, d'une pastille, d'un comprimé, d'une pilule, d'une boisson, d'une solution, d'un concentré, d'un sirop, d'une suspension, d'une ampoule liquide, ou d'une dispersion, et autres formes similaires. De manière préférée, la composition alimentaire selon l'invention est sous la forme de comprimés, poudre, capsules, pilules, et/ou boisson.

Par « excipient acceptable », on entend ici un composé susceptible d'améliorer la délivrance, la stabilité ou la biodisponibilité d'une composition (ici non thérapeutique), et qui peut être métabolisé et est non toxique pour un sujet auquel elle est administré. Des excipients préférés selon l'invention comprennent l'un quelconque des excipients communément utilisés dans les produits esthétiques, cosmétiques ou diététiques, tels que la cellulose microcristalline, le lactose, l'amidon, et la poudre de soja.

Selon un mode de réalisation préféré, ladite composition non thérapeutique comprend en outre au moins un agent capable de promouvoir et/ou accélérer la perte de poids, ou de ralentir et/ou atténuer la prise de poids . Ledit agent, qui peut être qualifié ici d'agent amincissant, peut être un agent anti-obésité, un agent hypolipémiant, un agent hypocholestérolémiant, voire un antidiabétique, tels que ceux décrits ci-dessus.

Un autre aspect de l'invention porte sur l'utilisation non thérapeutique de la composition amincissante de l'invention pour promouvoir et/ou accélérer la perte de poids, ou pour ralentir et/ou atténuer la prise de poids, chez un sujet.

En d'autres termes, l'invention porte sur une méthode non thérapeutique pour promouvoir et/ou accélérer la perte de poids, ou pour ralentir et/ou atténuer la prise de poids chez un sujet, ladite méthode comprenant une étape d'administration d'une quantité efficace de ladite composition audit sujet.

Selon un mode de réalisation préféré, ledit sujet est soumis à régime alimentaire normocalorique équilibré.

De manière préférée, ledit sujet est soumis audit régime alimentaire normocalorique équilibré, simultanément à l'administration de la composition non thérapeutique selon l'invention.

Selon un autre mode de réalisation préféré, l'administration de la composition non thérapeutique est effectuée avant et/ou pendant le ou les repas du sujet. Ce mode de réalisation peut être combiné ou non à un régime alimentaire normocalorique équilibré tel que décrit ci-dessus.

Le composé de l'invention peut également être administré en association avec un agent amincissant, et/ou à un régime alimentaire amincissant, de manière simultanée, séparée, ou étalée dans le temps. Ledit agent peut être un agent anti-obésité, un agent hypolipémiant, un agent hypocholestérolémiant, voire à un antidiabétique, tandis que le régime alimentaire peut être un régime alimentaire hypocalorique et/ou hypolipidique.

Lorsque le composé selon l'invention et l'agent amincissant sont administrés de manière séparée ou étalée dans le temps, ceux-ci peuvent être alors administrés dans des formes distinctes.

Ainsi, un autre objet de la présente description concerne un composé de formule (I) selon l'invention et un agent capable de promouvoir et/ou accélérer la perte de poids, ou de ralentir et/ou atténuer la prise de poids tel que décrit ci-dessus, en tant que préparation combinée pour une administration simultanée, séparée, ou étalée dans le temps. En d'autres termes, la présente description concerne une utilisation combinée du composé de formule (I) selon l'invention et d'un agent capable de promouvoir et/ou accélérer la perte de poids, ou de ralentier et/ou atténuer la prise de poids tel que décrit ci-dessus pour une administration simultanée, séparée, ou étalée dans le temps.

Un autre objet de l'invention est une méthode non thérapeutique pour promouvoir et/ou accélérer la perte de poids, ou pour ralentir et/ou atténuer la prise de poids chez un sujet, ladite méthode comprenant l'administration d'une quantité efficace du composé de formule (I) de l'invention audit sujet et la prise d'un régime alimentaire normocalorique équilibré chez ledit sujet, de manière simultanée, séparée, ou étalée dans le temps.

Un autre objet de la description est une méthode non thérapeutique pour promouvoir et/ou accélérer la perte de poids, ou pour ralentir et/ou atténuer la prise de poids chez un sujet, ladite méthode comprenant l'administration, audit sujet, d'une quantité efficace du composé de formule (I) selon l'invention et d'un composé amincissant tel que décrit ci-dessus, et la prise d'un régime alimentaire normocalorique équilibré chez ledit sujet, de manière simultanée, séparée, ou étalée dans le temps.

L'invention est illustrée par les exemples non limitatifs ci-après.

### DESCRIPTION DES FIGURES

**Figure 1****.** Expression des récepteurs CB1 dans des explants de foie de souris ob/ob traités 21h avec les molécules JM-00.246, JM-02.003, JM-01.1006, JM-00.266 ou JM-00.242.
**Figure 2****.** Variations d'AMPc dans les cellules HEK293T/17 transfectées par des plasmides pcDNA3.1-mCB1(50ng) et pGlo (100ng) et soumises à des concentrations croissantes de JM-02.003 **(2A),** JM-01.1006 **(2B)** et JM-00.266 **(2C)** en présence ou non d'AEA.
**Figure 3****.** Effet d'une injection i.p. de JM-02.003 (M2) ou JM-00.266 (M6) à 10 mg/kg sur la tolérance au glucose chez la souris sauvage (**3A et 3B**, respectivement) ou CB1R KO (**3C et 3D,** respectivement).
**Figure 4****.** Effet d'une injection i.p. de véhicule ou de JM-00.266 (M6) à 10 mg/kg sur la concentration en insuline plasmatique au cours d'un « *OGTT* » **(4A)** et sur la tolérance à l'insuline (« *ITT* »; **4B**) chez la souris sauvage.
**Figure 5****.** Effet d'une injection i.p. de véhicule, d'anandamide (AEA) ou d'AEA + JM-00.266 (M6) à 10 mg/kg sur le transit gastrointestinal chez la souris sauvage.
**Figure 6****.** Effet d'un traitement chronique de 30 jours par le SR141716 (i.e. Rimonabant), le composé JM-00.266 (M6) ou le véhicule sur l'anxiété et l'activité motrice des souris obèses déterminé par le test de champ ouvert (« *open field* ») **6A** : temps passé au centre (en secondes) ; **6B** : nombres de passages au centre ; **6C** : distance totale parcourue (en cm).
**Figure 7****.** Evolution de la prise alimentaire **(7A)** et du poids corporel **(7B)** au cours d'un traitement chronique de 30 jours par le composé JM-00.266 (M6) en comparaison au SR141716 (Rimonabant) et au véhicule. **(7C)** Evolution de la composition corporelle (EchoMRI) avant (J0) et à l'issue des traitements (J30).
**Figure 8****.** Effet d'un traitement chronique par SR141716 (Rimonabant) ou JM-00.266 (M6) à 10 mg/kg sur la glycémie basale **(8A)** et la tolérance au glucose de la souris obèse (« *OGTT* » 2g/Kg) (**8B** : avec rimonabant **et 8C** : avec M6).
**Figure 9****.** Effet d'un traitement chronique par le SR141716 (Rimonabant) **(9A)** ou JM-00.266 (M6) **(9B)** à 10 mg/kg sur la tolérance à l'insuline (« *ITT* ») chez la souris obèse.
**Figure 10****.** Variation de la masse corporelle de souris obèse soumise à un régime normolipidique (LF pour Low Fat) et traitée pendant 43 jours par le composé JM-00.266 (M6) (M6 + LF), en comparaison au véhicule en condition régime normolipidique (VEH + LF) ou au véhicule en condition hyperlipidique (VEH + HF pour High Fat).
**Figure 11****.** Effet d'un traitement de 43 jours par le composé JM-00.266 (M6) sur une souris obèse soumise à un régime normolipidique (M6 + LF) en comparaison au véhicule (VEH + LF), sur la tolérance au glucose.
**Figure 12****.** Variation de la masse corporelle de souris obèse traitée pendant 28 jours par le composé JM-00.266 (M6) administré par voie orale (10mg/kg) simultanément à la prise alimentaire, en comparaison au véhicule.
**Figure 13****.** Effet d'un traitement de 28 jours par le composé JM-00.266 (M6) administré par voie orale simultanément à la prise alimentaire sur une souris obèse, en comparaison au véhicule, sur la tolérance au glucose.

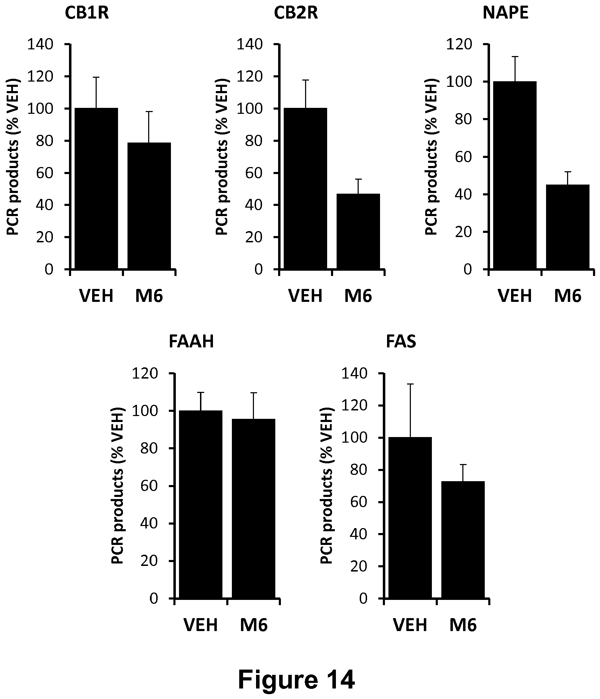

**Figure 14****.** Expression des récepteurs CB1R, CB2R, de l'enzyme de synthèse des endocannabinoides (NAPE), de l'enzyme de dégradation des endocannabinoides (FAAH), de la Fatty Acid Synthase (FAS) dans le foie de souris obèses traitées pendant 28j avec le composé JM-00.266 (M6) administré par voie orale simultanément à la prise alimentaire, en comparaison au véhicule (VEH).

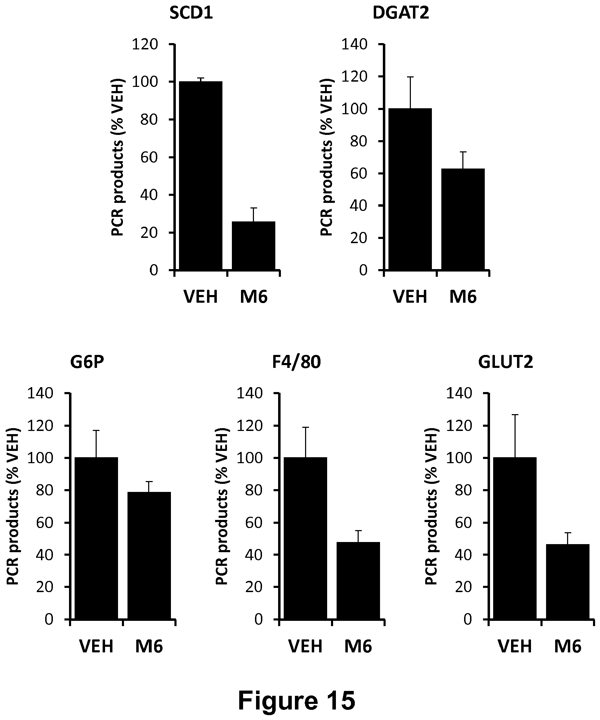

**Figure 15****.** Expression de la Stearoyl-CoA desaturase 1 (SCD-1), de la Acyl-coenzyme A:diacylglycerol acyltransferase 2 (DGAT2), de la Glucose-6-Phosphatase (G6P), du marqueur F4/80 des macrophages matures, et du transporteur du glucose GLUT2, dans le foie de souris obèses traitées pendant 28j avec le composé JM-00.266 (M6) administré par voie orale simultanément à la prise alimentaire, en comparaison au véhicule (VEH).

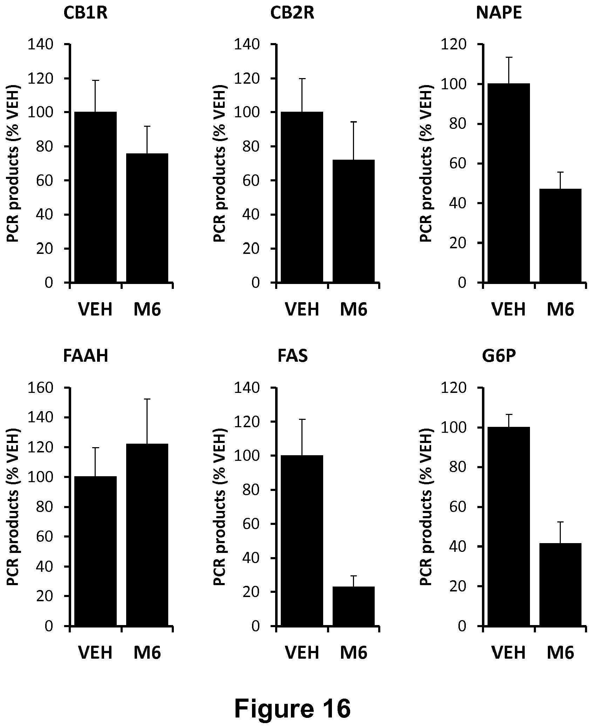

**Figure 16****.** Expression des récepteurs CB1R, CB2R, de l'enzyme de synthèse des endocannabinoides (NAPE), de l'enzyme de dégradation des endocannabinoides (FAAH), de la Fatty Acid Synthase (FAS), de la Glucose-6-Phosphatase (G6P) dans le tissu adipeux sous-cutané de souris obèses traitées pendant 28j avec le composé JM-00.266 (M6) administré par voie orale simultanément à la prise alimentaire, en comparaison au véhicule (VEH).

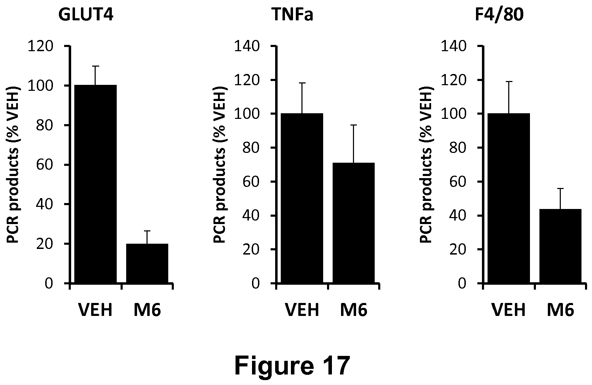

**Figure 17****.** Expression du transporteur de glucose GLUT4, du facteur α de nécrose tumorale (TNF-α pour Tumor necrosis factor-alpha), et du marqueur F4/80 des macrophages matures, dans des explants de tissu adipeux sous-cutané de souris obèses traitées pendant 28j avec le composé JM-00.266 (M6) administré par voie orale simultanément à la prise alimentaire, en comparaison au véhicule (VEH).

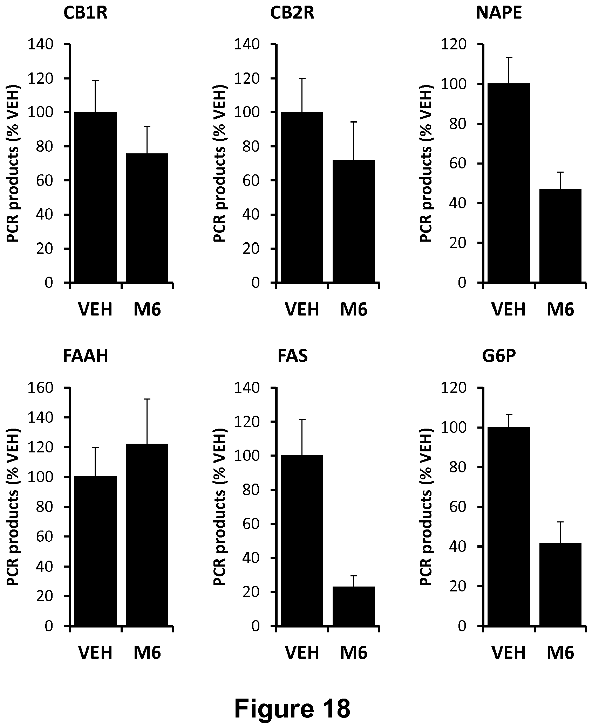

**Figure 18****.** Expression des récepteurs CB1R, CB2R, de l'enzyme de synthèse des endocannabinoides (NAPE), de l'enzyme de dégradation des endocannabinoides (FAAH), de la Fatty Acid Synthase (FAS) et de la Glucose-6-Phosphatase dans des explants de tissu adipeux viscéral de souris obèses traitées pendant 28j avec le composé JM-00.266 (M6) administré par voie orale simultanément à la prise alimentaire, en comparaison au véhicule (VEH).

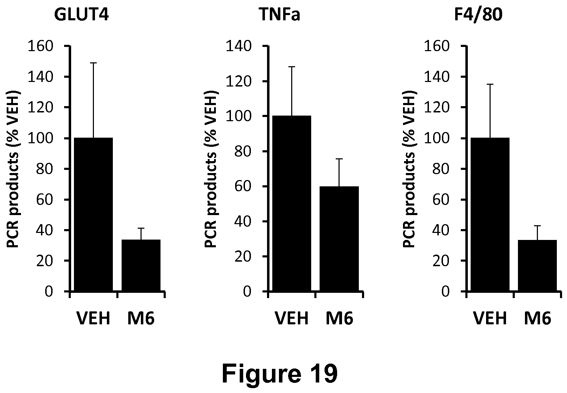

**Figure 19****.** Expression du transporteur du glucose GLUT4, du facteur α de nécrose tumorale (TNF-α pour Tumor necrosis factor-alpha), et du marqueur F4/80 des macrophages matures, dans des explants de tissu adipeux viscéral de souris obèses traitées pendant 28j avec le composé JM-00.266 (M6) administré par voie orale simultanément à la prise alimentaire, en comparaison au véhicule (VEH).

### EXEMPLES

### I. SYNTHESE DES COMPOSES SELON L'INVENTION

### 1. Matériels

Les points de fusion ont été déterminés au moyen d'un appareil Electrothermal IA9300, et sont reportés non corrigés.

Les spectres de 1H et 13C RMN ont été réalisés avec un spectromètre Bruker Avance 400 (400 MHz). Les déplacements chimiques (δ) sont exprimés en ppm avec le tétraméthylsilane comme témoin interne. Les représentations conventionnelles (s = singlet, d = doublet, t = triplet, q = quadruplet, sext = sextuplet, m = multiplet and b = broad) sont utilisées pour la description des spectres. Les constantes de couplage sont exprimées en Hertz (Hz).

L'analyse par spectrométrie de masse (SM) a été effectuée sur un spectromètre Waters Acquity UPLC System ZQ 2000 single quadrupole.

Les spectres infra-rouge sont réalisés sur un appareil Perkin-Elmer Paragon FTIR 1000 PC. Seules les bandes d'absorption caractéristiques sont présentées ; les valeurs du nombre d'onde sont exprimées en cm-1.

Le suivi des réactions est réalisé par chromatographie sur couche mince (CCM) sur gel de silice 60F-254 (5735 Merck), et les colonnes de purification chromatographique sur gel de silice 60 (70-230 Mesh, ASTM, Merck).

Tous les réactifs et solvants utilisés sont des produits commerciaux.

### 2. Synthèse de la 4-méthylthiobenzyl-4-méthylthiobenzaldimine (IV)

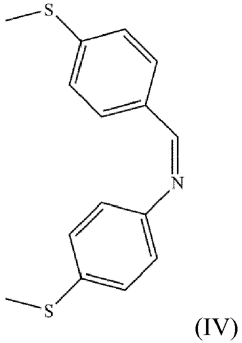

Dans 50mL de toluène, dissoudre 5,57g (36.6 mM) de 4-méthylthiobenzaldéhyde et 5,0g (35,92 mM) de 4-methylthioaniline. Ajouter 3g de sulfate de sodium anhydre, et porter au reflux du solvant, sous agitation, pendant 4 heures. Au bout de ce temps, le solvant est éliminé à l'évaporateur rotatif sous pression réduite. Le solide récupéré est trituré dans 10mL d'oxyde d'isopropyle et la suspension obtenue filtrée sur verre fritté. Recueillir ainsi 7,66g d'imine (rendement = 78%).

### Caractéristiques chimiques :

MP°C = 143-144 (Diisopropyle oxyde).
**¹H-RMN** (CDCl₃) : δ 2.51, s, 3H, 4-SCH3 ; : 2.54, s, 3H, 4'-SCH3 ; 7.18, d, 2H, H³H⁵, *J* = 6.7 Hz ; 7.29, d, 2H, H²H⁶ ; 7.30, d, 2H, H^{3'}H^{5'}, *J* = 8.4 Hz ; 7.80, d, 2H, H^{2'}H^{6'} ; 8.41, s, 1H, HC=N.
**SM** (ESI) m/z (%): 274[M+H]⁺
**IR** (KBr, cm⁻¹) : 1552.53 (v C=N).

### 3. Synthèse du chlorure d'acide de la phtaloylglycine

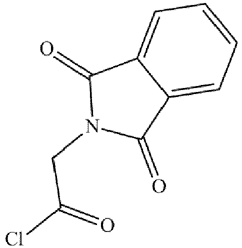

Dissoudre 2g (9,75 mM) de *N*-phtaloylglycine dans 20mL de chlorure de thionyle, et porter au reflux pendant 3 heures. Au bout de ce temps, le chlorure de thionyle est évaporé sous pression réduite à l'évaporateur rotatif. Le produit obtenu est repris trois fois par 50 mL de toluène, et soumis à chaque fois à une évaporation sous pression réduite. Au bout de la troisième évaporation, le produit obtenu est maintenu sous vide pendant 30 minutes, puis est repris par 20mL de dichloroéthane sec et conservé en l'état jusqu'à utilisation.

### 4. Synthèse de la trans-1,4-di-(4-méthylthiophényl)-3-N-phtaloyl-azétidine-2-one (composé IA = également dénommé ci-après JM-00.266 ou M6)

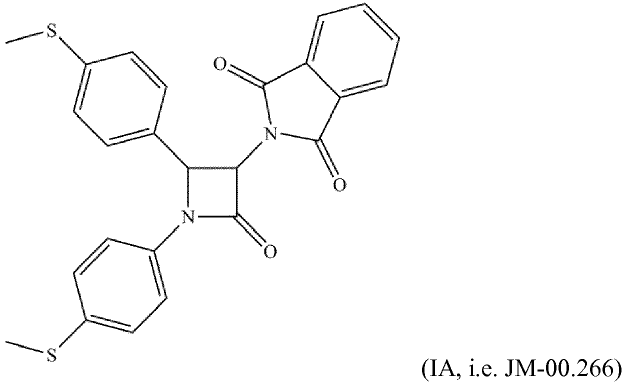

Dans un ballon de 250 mL, dissoudre 2,73g (10,0 mM) d'imine (IV) dans 50 mL de dichlorométhane sec. Ajouter 5 mL de triéthylamine et mettre l'ensemble sous agitation. Introduire alors lentement une solution de chlorure d'acide de la phtaloylglycine (9,75 mM) en maintenant le milieu à une température inférieure à 10°C. Une fois l'adjonction terminée, laisser le milieu revenir à la température ambiante, et maintenir ainsi en contrôlant l'évolution de la réaction par CCM. Au bout de 3 heures, la réaction n'évolue plus ; verser le milieu réactionnel dans 100 mL d'eau, et recueillir la phase organique à l'ampoule à décanter. Effectuer un autre lavage de celle-ci avec une quantité identique d'eau, puis la sécher sur du sulfate de sodium anhydre, filtrer et évaporer le solvant à sec. Le résidu obtenu est alors chromatographié sur colonne de silice en éluant par du dichlorométhane, ce qui permet d'obtenir 2,39g du composé (IA)
(R = 53%).

### Caractéristiques chimiques :

MP°C = 118-120 (Diethyl ether)
**¹H-RMN** (CDCl₃) : δ 2.44, s, 3H, CH₃ ; 2.49, s, 3H, CH₃ ; 5.26, d, 1H, Hₐ ; 5.33, d, 1H, H_{b} (*J*HₐH_{b} = 2.4Hz) ; 7.18, d, 2 arom.H, (J= 8.4Hz) ; 7.25-7.30, m, 4 arom.H ; 7.78, m, 2H, H4"-H5" ; 7.88, m, 2H, H3"-H6".
**¹³C-RMN** (100.6 MHz, CDCl) 16.45(CH₃) ; 16.50(CH₃) ; 60.99(Cb) ; 62.77(Ca) ; 118.15(2C) ; 123.85(C3"-C6") ; 126.63(2C) ; 127.00(2C) ; 127.91(2C) ; 131.65(C2"-C7") ; 132.10(C1) ; 134.14(C4-C1') ; 134.61(C4"-C5") ; 140.11(C4'); 161.75(C1"-C8") ; 166.65(Cc).
**SM** (ESI) m/z (%) : 461.6 [M+H]⁺
**IR** (KBr, cm⁻¹) : 3064, v CHₐᵣ ; 2974, 2922, 2835, ν CHₐₗᵢₚₕ ; 1759, 1714, ν C=O.

### 5. Autres composés testés

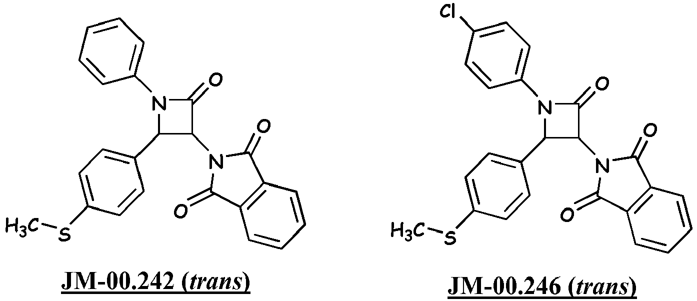

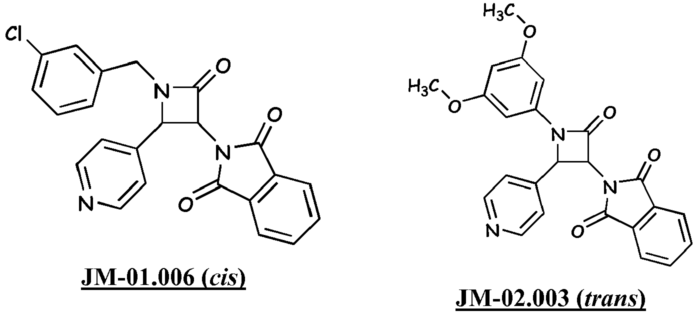

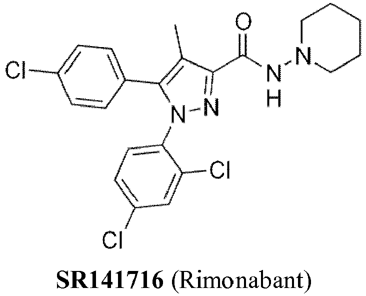

### 6. Voie de synthèse alternative de la trans-1,4-di-(4-méthylthiophényl)-3-N-phtaloyl-azétidine-2-one (composé IA = également dénommé ci-après JM-00.266 ou M6)

Une autre voie de synthèse du composé JM-00.266 a été développée. Elle consiste à mettre en contact l'imine (composé IV) avec la N-phtaloylglycine, et à générer le cétène *in situ* au moyen d'un agent de couplage, le dichlorophosphate de phényle en présence de triéthylamine comme accepteur de protons. L'intérêt de cette méthode est de s'affranchir de l'utilisation de chlorure de thionyle dont le maniement et l'élimination peuvent être délicats.

Selon un mode opératoire, 1,38g d'imine (composé IV) (5mM) sont mis en solution dans 20mL de dichlorométhane, sous agitation ; sont ajoutés alors 3mL de triéthylamine puis 1,128g de N-phtaloylglycine. Est introduit alors goutte à goutte dans le mélange, 1,5 mL (2,11g soit 10mM) de dichlorophosphate de phényle, et la réaction est laissée à température ambiante pendant 3h. Au bout de ce temps, le milieu réactionnel est lavé à l'eau, la phase organique est recueillie, séchée et concentrée sous pression réduite. Le résidu obtenu est chromatographié sur colonne de gel de silice en éluant par du dichlorométhane. Est ainsi recueilli 1,51g de composé IA (JM-00.266 *trans* ; l'isomère *cis* n'est pas présent dans le milieu réactionnel) avec un rendement de 66%.

### 7. Déprotection de la fonction amine à partir du trans-1,4-di-(4-méthylthiophényl)-3-N-phtaloyl-azétidine-2-one (composé IA = également dénommé ci-après JM-00.266 ou M6)

Par ailleurs, il a été réalisé la dé-protection de la fonction amine phtaloylée sur le composé JM-00.266. La libération de la fonction amine a été menée à bien avec succès par action de la méthylhydrazine dans le chlorure de méthylène.

Selon un mode opératoire, à 0,44g du composé IA JM-00.266 (0,955mM) en solution dans 20mL de dichlorométhane (DCM) est ajouté 0,1mL (2,18mM) de méthylhydrazine ; le mélange est laissé sous agitation d'abord à température ambiante puis en élevant lentement celle-ci jusqu'au reflux du DCM et en contrôlant l'évolution de la réaction par CCM. Lorsque celle-ci est achevée (4h), le milieu réactionnel est lavé à l'eau puis séché et concentré par évaporation à sec. Le résidu obtenu est chromatographié sur gel de silice au moyen d'une colonne courte (diamètre = 30mm ; longueur = 70mm) et en éluant par de l'acétate d'éthyle. 221mg de l'amine souhaitée sont ainsi recueillis (composé IF, également dénommé ci-après HR-0131 *trans*) avec un rendement allant de 70 à 81% avec un procédé de synthèse et de purification optimisé. 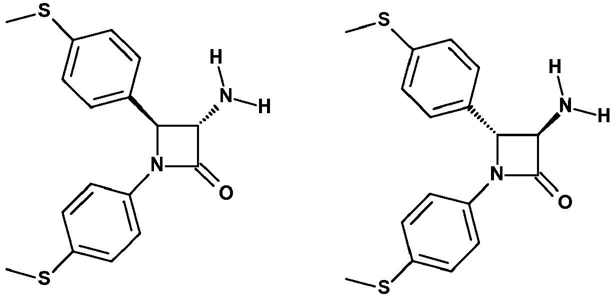 Structures des deux énantiomères du composé HR-0131 *trans*

### Caractéristiques physicochimiques du composé HR-0131 :

Formule brute : C₁₇H₁₈N₂S₂O ; masse moléculaire : 330.47 ; F = 134°C (AcOEt).
**IR** (KBr, cm⁻¹) : 3350-3061 (νCHₐᵣ) ; 2981-2835 (νCHₐₗᵢₚₕ) ; 1728 (vC=O).
**¹H-RMN,** δ(ppm), DMSO-d6 : 2.43, s, 3H, SCH₃(benzald) ; 2.49, s, 3H, SCH₃(anil) ; 3.66, 2H, NH₂ ; 3.92, d, 1H, (NCHlact) ; 4.73, d, 1H, (COCHlact) , J= 2.0 Hz ; 7.18-7.34, m, 8 Har.
**¹³C-RMN**, δ(ppm), DMSO-d6 : 14.73 ; 15.54 ; 65.41 ; 70.82 ; 117.75 ; 124.31 ; 126.41 ; 126.91 ; 127.52 ; 128.91 ; 132.41 ; 132.51 ; 133.08 ; 134.30 ; 135.01 ; 138.14 ; 168.80.
**SM** (ESI) m/z (%) : 331 [M+H]⁺

### 8. Synthèse de dérivés à partir de l'amine HR-0131.

A partir de l'amine préparée précédemment, il a été envisagé de synthétiser un dérivé homologue supérieur (HR-0133) du composé JM-00266. Ce dérivé résulte de la condensation de la phtaloylglycine sur HR-0131 et représente une structure dans laquelle a été introduit un espacement entre le cycle lactamique et le substituant phtaloyle. 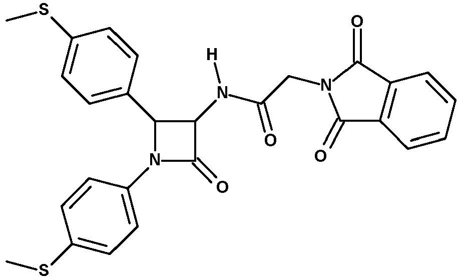 Structure du composé HR-0133 *trans*

Selon un mode opératoire, à une solution de 183 mg (0.55 mM) de HR-0131 dans 15 mL de dichlorométhane, sont ajoutés 144 mg (0.7 mM) de *N*-phtaloylglycine et 1 mL de triéthylamine. Sont ensuite ajoutés, goutte à goutte, 169 mg (0,8 mM) de dichlorophosphate de phényle, et le mélange est laissé sous agitation pendant 3H. Au bout de ce temps, le solvant est évaporé et le résidu obtenu est chromatographié sur colonne de silice en éluant par l'éther éthylique. 61 mg du produit désiré (HR-0133 trans) sont obtenus (R=21%, ce rendement pouvant être amélioré en optimisant le procédé de synthèse et de purification).

### Caractéristiques physicochimiques du composé HR-0133 :

Formule brute : C₂₇H₂₃N₃S₂O₄ ; masse moléculaire : 517.63 ; F = 190-192°C (iPrOiPr). **IR** (KBr, cm⁻¹) : 3348 (νCHₐᵣ) ; 2998-2918 (νCHₐₗᵢₚₕ) ; 1728, 1693 (vC=O).
**¹H-RMN,** δ(ppm), DMSO-d6 : 2.43, s, 3H, SCH₃(benzald) ; 2.49, s, 3H,S CH₃(anil) ; 4.37, s, 2H, CH₂ ; 4.72, dd, 1H, (COCHlact), J = 2.4 Hz, J = 7.6 Hz ; 5.05, d, 1H, (NCHlact), J = 2.4 Hz ; 7.18, d, 2H, H₂H₆benzald, J = 8.8 Hz ; 7.23, d, 2H, H₃H₅benzald, J = 8.8 Hz ; 7.29, d, 2H, H₃H₅anil, J = 8.4 Hz ; 7.40, d, 2H, H₂H₆anil, J = 8.4 Hz ; 7.90-7.98, m, 4H, FtₐᵣH, 9.20, d, 1H, NH, J = 7.6 Hz.
**¹³C-RMN**, δ(ppm), DMSO-d6 : 14.62; 15.39 ; 40.33 ; 61.49 ; 65.08 ; 117.86(2C) ; 123.44(2C) ; 126.34(2C) ; 127.35(2C) ; 127.39 ; 131.88 ; 133.09(2C) ; 133.19(2C) ; 134.40 ; 134.80(2C) ; 141.00 ; 163.98 ; 166.98 ; 167.59(2C).
**SM** (ESI) m/z (%) : 518 [M+H]⁺.

### II. ACTIVITÉ BIOLOGIQUE DES COMPOSÉS SYNTHÉSISÉS

### 1. MATERIEL ET METHODES

### 1.1. Etudes in vitro

### 1.1.1. Culture d'explants de foie

Le foie des souris a été perfusé *in situ,* sous anesthésie au pentobarbital sodique (50 mg/kg), avec du milieu de Hank's (pH 7,4) saturé en oxygène. Ensuite, le foie a été tranché grâce à un slicer Brendel/Vitron (Tucson, AZ, USA) dans le même milieu. Les coupes de foie (environ 200 µm) ont ensuite été incubées 21h dans du milieu William's E (WME) oxygéné et supplémenté en sérum de veau fœtal désactivé (10%) et cocktail d'antibiotiques/antifongiques (1%) sous atmosphère contrôlée (5% CO2), auquel est ajouté soit l'antagoniste à tester soit le véhicule.

### 1.1.2. Expression génique

L'extraction des ARN messagers (ARNm) totaux a été réalisée avec du Tri-Reagent (Euromedex, France) et la synthèse consécutive d'ADN complémentaire (ADNc) a été réalisée à partir d'1 µg d'ARNm avec le kit Bio-Rad iScripttm Reverse Transcription super mix (Bio-Rad, France).

L'expression des gènes a été évaluée par RT-PCR (Reverse Transcription - Polymerase Chain Reaction) semi-quantitative en temps réel. Les amorces utilisées ont été dessinées à l'aide du logiciel Primer3Plus (http://www.bioinformatics.nl/cgi-bin/primer3plus/primer3plus.cgi) et synthétisées par MWG-Biotech (TATABox Binding Protein : Sens acggcacaggacttactcca, antisens gctgtctttgttgctcttccaa ; CB1R : Sens ccgcaaagatagtcccaatg, antisens aaccccacccagtttgaac). La semi-quantification de l'expression des gènes a été obtenue en tenant compte de l'efficacité de chaque PCR et après standardisation avec le gène rapporteur TATA Box Binding protein.

### 1.1.3. Test GloSensor cAMP

Des cellules HEK293T/17 (ATCC) ont été mises en culture dans du DMEM 10% SVF, puis ensemencées à 30000 cellules/puits dans des plaques plaques 96 puits. Après 24h, les cellules ont été soumises à une transfection transitoire par Fugene HD (Promega) par des plasmides pcDNA3.1-mCB1(50ng) et pGlo (100ng) avec ou sans PTX (Pertussis toxin). Pour les essais contrôles les cellules ont été transfectées avec des vecteurs vides pcDNA3 (VV) et pGlo avec ou sans PTX. A 48h (24h post-transfection), les cellules ont été chargées pendant 2h avec le GloReagent à 2% dans un milieu CO2-independant à 10% SVF (80µL/puits).

Les cellules ont ensuite été traitées par ajout à t=0, de 10µL/puits de Forskolin (FSK) 1µM final, afin d'augmenter la concentration basale d'AMPc. La cinétique d'apparition de l'AMPc a été contrôlée pendant 10 minutes. A t=10, les molécules à tester ont été ajoutées et les variations d'AMPc mesurées pendant 20 minutes. Le signal lumineux a été mesuré en RLU et est exprimé en % de réponse par rapport au signal lu à t=10min (FSK1µM). Les courbes sigmoïdales ont été obtenues par la mesure à t=10min après addition de la molécule à tester (donc t=20 min total) du pourcentage de signal lumineux en fonction de la concentration en molécule. La régression 4PL a été réalisée à l'aide du logiciel Sigma Plot et a permis d'obtenir un EC50 (1 expérience, n=3).

### 1.2. Etudes in vivo à court terme : tests en aigu.

### 1.2.1. Transit Gastrointestinal

Le transit à travers l'estomac et l'intestin a été mesuré via l'administration orale de charbon végétal en suspension dans la gomme arabique utilisé ici comme marqueur non absorbable. Brièvement, suite à un jeûne court, des souris C57BL/6 ont été injectées intrapéritonéalement (i.p.) avec de l'anandamide (10 mg/kg) en présence ou non de la molécule d'intérêt JM-00.266 (i.e. composé IA) (10 mg/kg) avant l'administration orale de charbon. 25 minutes après, les animaux ont été sacrifiés par dislocation cervicale afin de retirer entièrement l'intestin. La distance entre le début du pylore et la localisation du bolus de charbon a été mesurée.

### 1.2.2. Test oral de tolérance au glucose (« OGTT »)

Afin d'évaluer les effets à court terme des molécules analogues au Rimonabant, ainsi que leur sélectivité pour les récepteurs CB1R, des souris C57BL/6 sauvages et des souris CB1R -/- ont subi un test oral de tolérance au glucose (« *OGTT »* à 2 g/kg) 10 min après l'injection i.p. de véhicule ou de JM-02.003 ou JM-00.266 (i.e. composé IA) (10 mg/kg). La glycémie a été mesurée aux temps t=0, t=15 min, t=30 min, t=45 min, t=60 min, t=90 min, et t=120 min post-administration orale. de glucose à l'aide d'un lecteur glycémique Contour®TS (Réf. 81574201, Bayer HealthCare) et de bandelettes réactives (Réf. 81574274, Bayer HealthCare).

### 1.2.3. Test de tolérance à l'insuline (« ITT »)

L'effet à court terme de la molécule JM-00.266 (i.e. composé IA) sur la sensibilité à l'insuline a été mesuré au cours d'un test de tolérance à l'insuline. Pour cela, des souris C57BL/6 sauvages ont été injectées en intrapéritonéal avec de l'insuline à action rapide (0.5 UI/kg; réf. YT60088, Actrapid®) 10 min après l'injection i.p. de véhicule ou de JM-00.266 (i.e. composé IA) (10 mg/kg). La glycémie a été mesurée aux temps t=0, t=15 min, t=30 min, t=45 min, t=60 min, t=90 min, et t=120 min post-injection i.p. d'insuline à l'aide d'un lecteur glycémique.

### 1.2.4. Insuline plasmatique

Le dosage de l'insuline plasmatique a été réalisé à l'aide du kit Insuline de souris ELISA ALPCOTM (Réf. AKRIN-011T, ALPCO Diagnostics) selon les instructions du fournisseur. Les prélèvements sanguins ont été récupérés lors d'un « *OGTT* » à t=0, t=30, t=60, t=120 min post-administration du bolus oral de glucose (2 g/kg).

### 1.3. Etudes in vivo à long terme : administration chronique

### 1.3.1. Régime, prise alimentaire et composition corporelle des animaux

Afin de déterminer les effets à long terme des molécules d'intérêts, des souris C57BL/6 rendues obèses grâce à un régime riche en sucre et en graisse (High Sucrose High Fat ; HSHF : 30% de lard, 33,5% de glucides ; réf. E15126-34,; réf. E15126-34,, SSNIFF, Soest, Germany) pendant 20 semaines. Ces souris ont ensuite reçu quotidiennement une injection i.p de Rimonabant, JM-00.266 (i.e. composé IA) ou de véhicule sur une durée de 30 jours. Parallèlement, le poids et la prise alimentaire ont été surveillés tous les deux jours suivant le début du traitement.

L'effet du traitement à long terme sur la composition corporelle a été mesuré à l'aide d'un scanner EchoMRI™ permettant l'analyse non invasive de la masse grasse, la masse maigre et de la composition en fluides corporels par résonnance magnétique nucléaire (RMN) sur l'animal vivant sans anesthésie.

### 1.3.2. Etude comportementale : Test de champ ouvert ou « open field »

L'activité locomotrice des souris a été mesurée à l'issue du traitement chronique avec le Rimonabant ou la molécule JM-00.266 (i.e. composé IA) par un système de suivi par infrarouge. Pour cela, les animaux ont été placés de façon individuelle dans des boîtes en plexiglas de 43 x 43 cm (MED associates) durant 20 min. Deux séries de 16 faisceaux infrarouges modulés par impulsions ont été placées sur des parois opposées espacées de 2,5 cm pour enregistrer les mouvements ambulatoires X-Y à une résolution de 100 ms. Le centre a été définie comme un carré central de 32×32 cm. En plus des informations sur l'activité locomotrice, ce test permet de prédire une activité de type anxiolytique en réponse à la nouveauté ou à un environnement anxiogène. Les variables mesurées dans le champ ouvert sont l'activité ambulatoire totale (en cm), le nombre d'entrées et le temps passé dans l'aire centrale ainsi que la distance parcourue dans le centre divisée par la distance totale parcourue.

### 1.3.3. Dosages plasmatiques

Le cholestérol total, les triglycérides et les marqueurs hépatiques ont été dosés par un automate Analyzer Dimension Vista (Siemens, Saint-Denis, France) à l'aide des réactifs appropriés.

### 1.3.4. Test oral de tolérance au glucose et test de tolérance à l'insuline

Afin d'évaluer les effets du traitement à long terme sur le contrôle glycémique, la tolérance au glucose (« *OGTT* ») ainsi que la sensibilité à l'insuline (« *ITT* ») ont été évaluées en pré- et post-traitement. Ainsi, pour l'« *OGTT* » les souris ont été gavées avec du glucose (2 g/kg), et pour l' « *ITT* » les souris ont reçu une injection i.p. d'insuline (0.5 UI/kg). Dans les deux cas, la glycémie a été mesurée aux temps t=0, t=15 min, t=30 min, t=45 min, t=60 min, t=90 min, et t=120 min post-ingestion de glucose à l'aide d'un lecteur glycémique Contour®TS (Réf. 81574201, Bayer HealthCare) et de bandelettes réactives (Réf. 81574274, Bayer HealthCare).

### 1.4 Etude in vivo à long terme : administration du composé IA en association avec une diminution de l'apport énergétique sur la souris obèse

### 1.4.1 Régime hyperlipidique, prise alimentaire associée à un régime normolipidique et masse corporelle des animaux

Afin de déterminer les effets à long terme du composé d'intérêt JM-00.266 (i.e. composé IA), des souris C57BL/6 ont été rendues obèses grâce à un régime hyperlipidique (30% de lard, 33,5% de glucides ; réf. E15126-34, SSNIFF, Soest, Germany) pendant 15 semaines. Les souris ont ensuite été soumises à un régime normolipidique (5% de lipides ; Standard Diet AO4; UAR, Epinay-sur-Orge, France) et ont reçu quotidiennement, en milieu de journée, une dose orale de JM-00.266 (i.e. composé IA) ou de véhicule sur une durée de 43 jours. Le poids des animaux a été mesuré tous les deux jours à partir du début du traitement.

### 1.4.2 Test de tolérance au glucose

Afin d'évaluer les effets du traitement à long terme sur le contrôle glycémique, la tolérance au glucose a été évaluée à la fin de la période de traitement. Les souris ont subi une injection intrapéritonéale de glucose (2 g/kg) puis la glycémie a été mesurée aux temps t=0, t=15 min, t=30 min, t=45 min, t=60 min, t=90 min, et t=120 min post-injection de glucose à l'aide d'un lecteur glycémique My Life Pura (Ypsomed, Paris, France).

### 1.5 Etude in vivo à long terme : administration du composé IA simultanée à la prise alimentaire chez la souris obèse maintenue à un régime hyperlipidique

Le choix de cette approche se justifie par le fait que les résultats antérieurs montrent que lorsque l'administration de M6 précède une charge en glucose, la tolérance au sucre est très nettement améliorée.

### 1.5.1 Régime hyperlipidique, administration du composé simultanée à la prise alimentaire et masse corporelle des animaux

Des souris C57BL/6 ont été rendues obèses grâce à un régime hyperlipidique (35% de lard, 25,3% de glucides ; réf. E15742-34, SSNIFF, Soest, Germany) pendant 15 semaines. Les souris maintenues au même régime ont ensuite reçu quotidiennement une dose orale de JM-00.266 (i.e. composé IA) ou de véhicule incorporée à l'alimentation sur une durée de 43 jours. Le poids des animaux a été mesuré tous les deux jours à partir du début du traitement.

### 1.5.2 Test de tolérance au glucose

Afin d'évaluer les effets du traitement à long terme sur le contrôle glycémique, la tolérance au glucose a été évaluée à la fin de la période de traitement. Les souris ont subi une injection intrapéritonéale de glucose (2 g/kg) puis la glycémie a été mesurée aux temps t=0, t=15 min, t=30 min, t=45 min, t=60 min, t=90 min, et t=120 min post-injection de glucose à l'aide d'un lecteur glycémique My Life Pura (Ypsomed, Paris, France).

### 1.5.3 Expression génique

L'extraction des ARN messagers (ARNm) totaux a été réalisée avec du Tri-Reagent (Euromedex, France) et la synthèse consécutive d'ADN complémentaire (ADNc) a été réalisée à partir d'1 µg d'ARNm avec le kit Bio-Rad iScripttm Reverse Transcription super mix (Bio-Rad, France). L'expression des gènes a été évaluée par RT-PCR (Reverse Transcription-Polymerase Chain Reaction) semi-quantitative en temps réel. Les amorces utilisées décrites ci-dessous ont été sélectionnées à l'aide du logiciel Primer3Plus (http://www.bioinformatics.nl/cgi- 25 bin/primer3plus/primer3plus.cgi) et synthétisées par MWG-Biotech. La semi-quantification de l'expression des gènes a été obtenue en tenant compte de l'efficacité de chaque PCR et standardisation avec le gène rapporteur TATABox- Binding protein (TBP).

### Amorces utilisées :

| Gene | SEQ ID N° | 5'-sense primer-3' | SEQ ID N° | 5'-antisense primer-3' |
|---|---|---|---|---|
| TPB | 1 | acggcacaggacttactcca | 2 | gctgtctttgttgctcttccaa |
| CB1R | 3 | ccgcaaagatagtcccaatg | 4 | aaccccacccagtttgaac |
| CB2R | 5 | caaaggaggaagtgcttggt | 6 | tggagagatcggcttatgttg |
| F4/80 | 7 | tgacaaccagacggcttgtg | 8 | gcaggcgaggaaaagatagtgt |
| FAAH | 9 | ggaccttgctcccctttct | 10 | cctgctgggctgtcacata |
| FAS | 11 | ggctgcagtgaatgaatttg | 12 | ttcgtacctccttggcaaac |
| G6P | 13 | tggcctggcttattgtacct | 14 | gtgctaagaggaagacccga |
| GLUT2 | 15 | ctcttcaccaactggccct | 16 | cagcagataggccaagtagga |
| GLUT4 | 17 | gatgccgtcgggtttccagca | 18 | tgttccagtcactcgctgccg |
| DGAT2 | 19 | agccctccaagacatcttctct | 20 | tgcagctgtttttccacct |
| NAPE-PLD | 21 | ctcgatatctgcgtggaaca | 22 | ctgaattctggcgctttctc |
| SCD1 | 23 | ccggagaccccttagatcga | 24 | tagcctgtaaaagatttctgcaaacc |
| TNF-a | 25 | cggggtgatcggtccccaaag | 26 | tggtttgctacgacgtgggct |

L'impact du traitement sur l'activité du système endocannabinoïde est évaluée en mesurant l'expression génique 1) des récepteurs CB1R et CB2R 2) de l'enzyme de synthèse des endocannabinoides, la N-acyl phosphatidylethanolamine phospholipase D (NAPE-PLD) et 3) de l'enzyme de dégradation des endocannabinoides, la Fatty acid amide hydrolase (FAAH).

La Fatty acid synthase (FAS), Stearoyl- CoA desaturase 1 (SCD-1) et la Glycerol-Phosphate acyl-transferase (GPAT2) sont des enzymes dont les variations d'expression traduisent l'activité lipogénique.

La Glucose-6-phosphatase (G6P) et les transporteurs du glucose GLUT2 et GLUT4 dans le foie sont utilisés ici comme marqueurs de la néoglucogenèse.

F4/80 est un marqueur des macrophages matures.

Le Tumour necrosis factor-alpha (TNF-a) est une cytokine pro-inflammatoire intervenant dans la régulation de nombreux processus biologiques comme les fonctions immunitaires, la différentiation cellulaire ou le métabolisme énergétique.

### 2. RESULTATS

### 2.1. Expérimentations in vitro et in vivo à court terme (injections aigues) sur les composés JM-00.246, JM-02.003, JM-01.1006, JM-00.242 et JM-00.266 (i.e. composé IA).

### 2.1.1. Effets des molécules candidates JM-00.246, JM-02.003, JM-01.006, JM-00.266 (i.e. composé IA) et JM-00.242 sur l'expression hépatique du CB1R chez la souris Ob/Ob

Des études précédemment menées au laboratoire ont indiqué qu'il était possible de moduler l'expression des CB1R dans des explants de foie mis en culture en présence d'agonistes ou d'antagonistes (Jourdan *et al.* 2012). Ainsi, l'expression des CB1R dans des explants de foie est réduite suite à un traitement par le SR141716 (i.e. Rimonabant). Par conséquent, ce modèle *in vitro* a été utilisé ici pour présélectionner les molécules candidates sur la base de leur capacité à modifier l'expression du CB1R.

Sur la base de ces précédents résultats obtenus avec le SR141716 (i.e. Rimonabant), la capacité de chacun des composés à diminuer l'expression des CB1R a été testée dans le même modèle d'explants de foie.

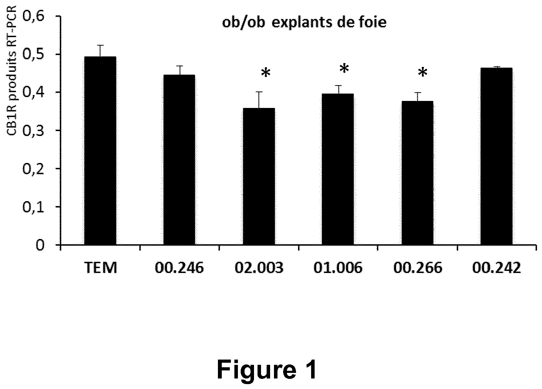

Seules les molécules JM-02.003, JM-01.006 et JM-00.266 (i.e. composé IA) ont provoqué une diminution significative de l'expression des CB1R hépatiques c'est à dire un effet comparable à celui observé dans l'étude précédente avec le SR141716 (i.e. Rimonabant) (Figure 1). Les molécules JM-00.246 et JM-00.242 n'ont pas été retenues pour la suite de l'étude.

### 2.1.2. Effets des molécules présélectionnées JM-02.003, JM-01.1006 et JM-00.266 sur l'activité du récepteur CB1

Les capacités de JM-02.003, JM-01.006 et JM-00.266 (i.e. composé IA) à antagoniser les CB1R ont été testées dans un modèle de cellules transfectées avec le CB1R en mesurant les variations d'AMPc (GloSensor cAMP assay).

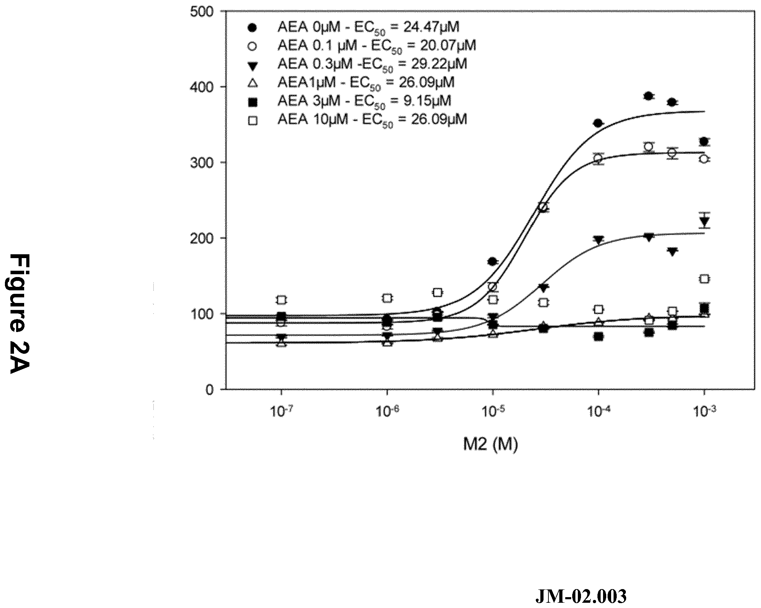

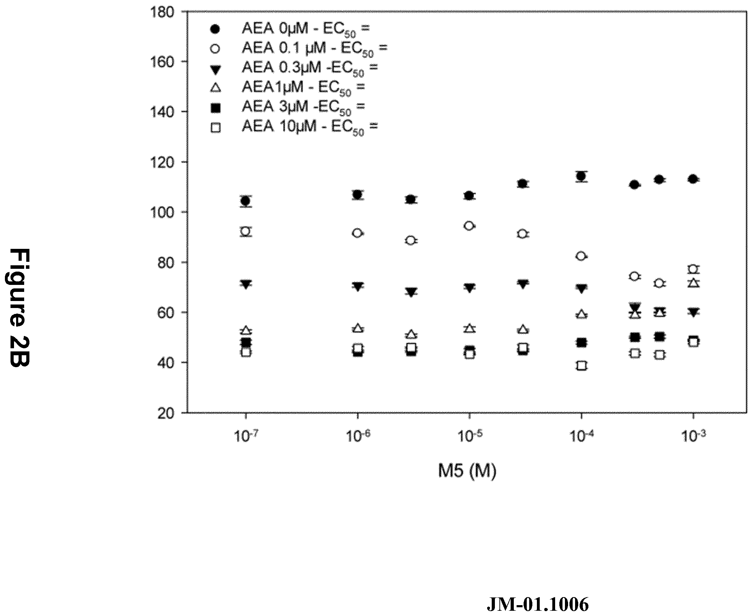

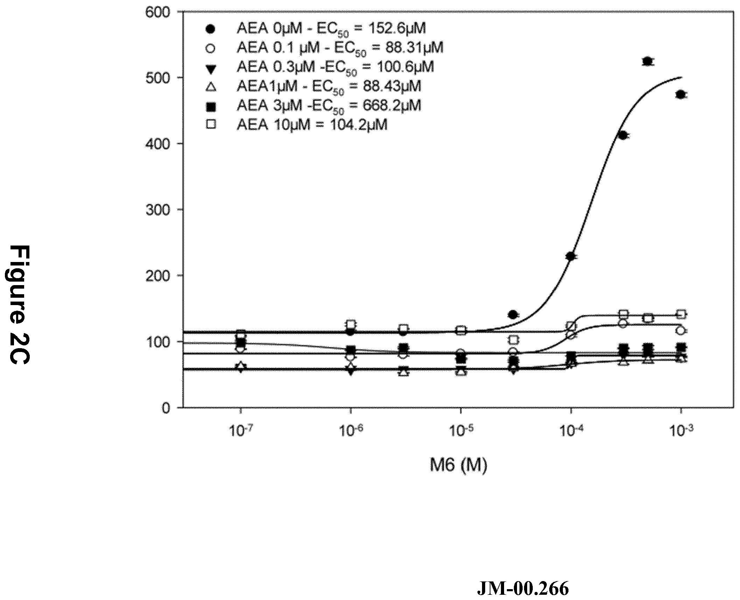

Dans un premier temps, il a été vérifié en utilisant ce modèle *in vitro* que l'activation des récepteurs par un agoniste, ici l'AEA, conduit bien à une diminution de la concentration intracellulaire d'AMPc (données non présentées). Les résultats indiquent que les concentrations d'AMPc sont augmentées en absence d'agoniste (AEA) dans les cellules traitées par JM-02.003 et JM-00.266 (i.e. composé IA) alors que JM-01.006 est sans effet (Figure 2). Ces données confirment que seules les molécules JM-02.003 et JM-00.266 (i.e. composé IA) sont des ligands des CB1R et exercent un effet agoniste inverse sur le récepteur. La molécule JM-01.1006 n'a donc pas été retenue pour la suite de l'étude.

### 2.1.3. Effets d'un traitement aigu par les molécules JM-02.003 et JM-00.266 (i.e. composé IA) sur la tolérance au glucose de la souris C57B1/6J sauvage.

Afin d'évaluer les effets *in vivo* des molécules retenues, les Inventeurs ont cherché à déterminer si une seule injection i.p. pouvait moduler le métabolisme glucidique de la souris. En effet, des travaux récents ont montré que l'activation des CB1R en réponse à une seule injection i.p. d'AEA (agoniste CB1R) altère la tolérance au glucose et la résistance à l'insuline (Liu et al., 2012).

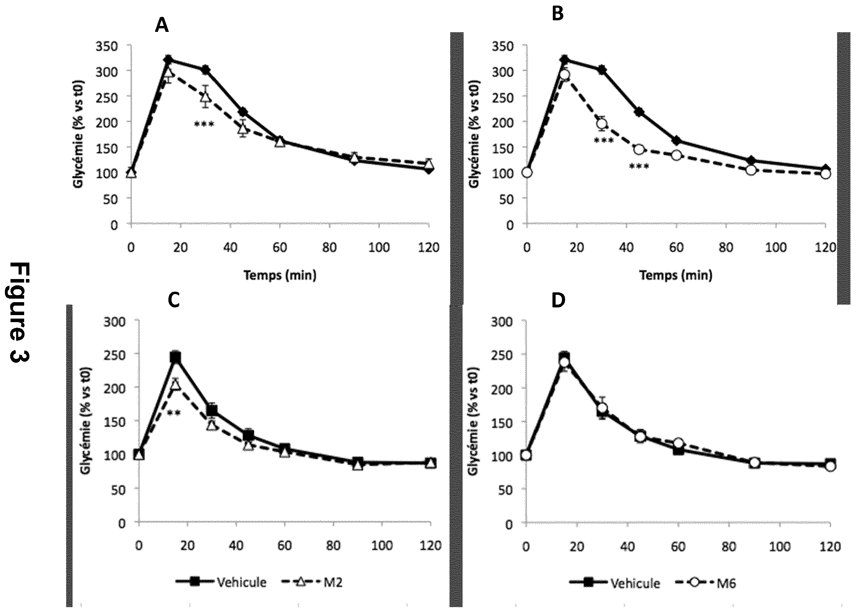

Pour cela, des souris C57Bl/6J sauvages ont reçu une injection i.p. d'antagoniste JM-02.003 ou JM-00.266 (i.e. composé IA) à 10 mg/kg 10 min avant une charge orale en glucose à 2g/kg. Les présents résultats montrent que l'injection i.p. de ces 2 molécules améliore la tolérance au glucose par rapport aux souris ayant reçu le véhicule (Figure 3A et B).

Lorsque ces expériences sont répétées chez les souris CB1R KO, on observe que l'amélioration de la tolérance au glucose est abrogée chez la souris traitées par JM-00.266 (i.e. composé IA) alors qu'elle persiste avec JM-02.003 (Figure 3C et D). Ceci confirme que seule la molécule JM-00.266 (i.e. composé IA) exerce un effet agoniste inverse CB1R spécifique. La molécule JM-02.003 n'a donc pas été retenue pour la suite de l'étude.

### 2.1.4. Effets d'un traitement aigu par JM-00.266 (i.e. composé IA) sur la sensibilité et la production d'insuline de la souris C57B1/6J sauvage.

Afin de savoir si l'amélioration du contrôle glycémique observée en réponse à l'injection i.p. de JM-00.266 (i.e. composé IA) est potentiellement due à une augmentation de la sécrétion d'insuline et/ou à une meilleure sensibilité à l'insuline, un dosage d'insuline plasmatique a été réalisé suite à l'administration orale du bolus de glucose ainsi qu'un test de tolérance à l'insuline (ITT).

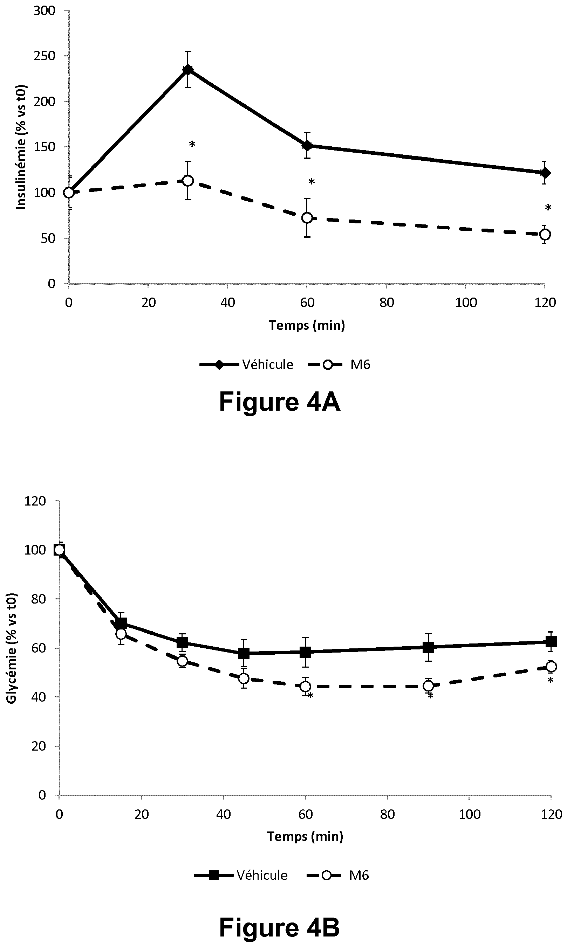

Les résultats montrent que la production d'insuline induite par l'administration de glucose n'est pas stimulée par JM-00.266 (Figure 4A). Au contraire, l'insulinémie à t=30 min est plus faible chez la souris traitée au JM-00.266 (i.e. composé IA) suggérant une amélioration des capacités d'utilisation du glucose. L' « *ITT* » révèle également que l'insuline exerce un effet plus puissant sur la clairance du glucose plasmatique chez les animaux préalablement traités par JM-00.266 (i.e. composé IA) par rapport aux animaux témoins (Figure 4B).

Ces données suggèrent que la molécule JM-00.266 (i.e. composé IA) améliore la tolérance au glucose en augmentant la sensibilité à l'insuline.

### 2.1.5. Effets d'un traitement aigu par JM-00.266 (i.e. composé IA) sur le transit gastro-intestinal de la souris C57B1/6J sauvage.

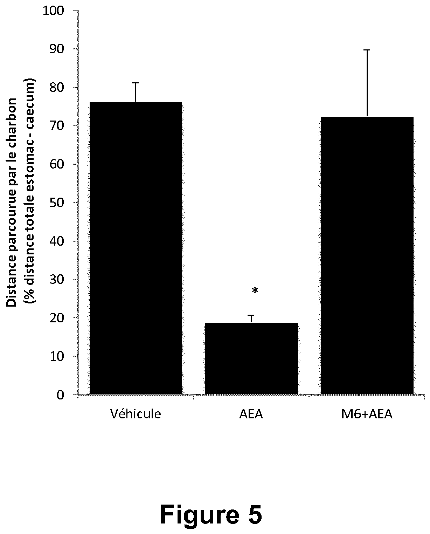

Il est clairement démontré dans la littérature que l'activation des CB1R inhibe puissamment la motilité gastro-intestinale (Di Marzo et al., 2008). Sur cette base, les Inventeurs ont cherché à savoir si la molécule JM-00.266 (i.e. composé IA) était capable d'améliorer la gastroparésie induite par un agoniste CB1R (AEA) en mesurant la progression *in vivo* d'un bolus de charbon non absorbable administré par gavage le long du tractus digestif. Dans le modèle expérimental testé, le transit est, comme attendu, fortement inhibé par l'AEA. De façon intéressante, l'injection préalable de JM-00.266 (i.e. composé IA), abroge totalement l'effet de l'agoniste et normalise le transit gastro-intestinal (Figure 5). Ces données indiquent d'une part que le tractus digestif est une cible de JM-00.266 (i.e. composé IA) et d'autre part que cette molécule est capable d'annuler les effets d'un agoniste sur les CB1R, c'est-à-dire d'exercer un effet antagoniste sur le récepteur.

### 2.2. Expérimentations in vivo à long terme (injections chroniques) chez la souris obèse avec le composé JM-00.266.

Afin de déterminer les effets à long terme du composé JM-00.266 (i.e. composé IA), des souris C57BL/6 ont été rendues obèses grâce à un régime riche en sucre et en graisse administré pendant 20 semaines. Ces souris ont ensuite reçu quotidiennement une injection i.p de SR141716 (i.e. Rimonabant) (10 mg/kg), JM-00.266 (i.e. composé IA) (10 mg/kg) ou de véhicule sur une durée de 30 jours.

### 2.2.1. Effets du traitement chronique par le composé JM-00.266 (i.e. composé IA) sur les marqueurs de souffrance hépatique

Les enzymes hépatiques, alanine aminotransférase (ALT), aspartate aminotransférase (AST), alkaline phosphatase, intestinale (ALPI), les gamma GT et la bilirubine totale sont des marqueurs de souffrance cellulaire. Ces marqueurs ont été mesurés dans le plasma afin de détecter une éventuelle toxicité hépatique du composé JM-00.266 (i.e. composé IA).

**Tableau 1. Concentration plasmatique en marqueurs hépatiques à l'issue d'un traitement chronique de 30 jours par le SR141716 (Rimonabant) ou le composé JM-00.266 (composé IA, également dénommé M6) en comparaison au véhicule (Témoin). ALT : alanine aminotransférase ; AST : aspartate aminotransférase, ALPI : alkaline phosphatase, intestinale.**

| | **Témoin** | **SR141716** | **JM-00.266** |
|---|---|---|---|
| Gamma GT (U/L) | <3 | <3 | <3 |
| ALT (U/L) | 62,0 ± 24,9 | 21,6 ± 2,3* | 34,3 ± 10,5 |
| AST (U/L) | 131,2 ± 47,0 | 46,4 ± 31* | 60,8 ± 13,4 |
| ALPI | 52,7 ± 7,8 | 37,4 ± 2,4 | 47,3 ± 3,6 |
| Bilirubine Totale (µmol/L) | <2 | <2 | <2 |

Les résultats présentés dans le Tableau 1 montrent qu'un traitement chronique de 30 jours avec le SR141716 (i.e. Rimonabant) ou le JM-00.266 (i.e. composé IA) n'induit pas d'augmentation des marqueurs de souffrance hépatiques par rapport au témoin. Au contraire, les concentrations d'ALT et d'AST détectées dans le plasma des souris obèses sont diminuées significativement par le SR141716 (i.e. Rimonabant) pendant que la même tendance est observée avec JM-00.266 (i.e. composé IA).

En conclusion, les résultats suggèrent non seulement que l'administration chronique du composé JM-00.266 (i.e. composé IA) n'induit aucune toxicité hépatique mais également qu'il améliorerait la souffrance cellulaire induite par l'obésité.

### 2.2.2. Effets du traitement chronique par le composé JM-00.266 (i.e. composé IA) sur certains paramètres comportementaux liés à l'activation des CB1R centraux.

Le test de champ ouvert (open field) consiste à mesurer, par un système de faisceaux infrarouges, les déplacements de l'animal dans une enceinte éclairée représentant un environnement stressant. Les variables mesurées dans l'open field sont l'activité ambulatoire totale, le nombre d'entrées et le temps passé dans l'aire centrale. Ce test permet, en plus de mesurer l'activité locomotrice, de renseigner sur l'état d'anxiété de l'animal.

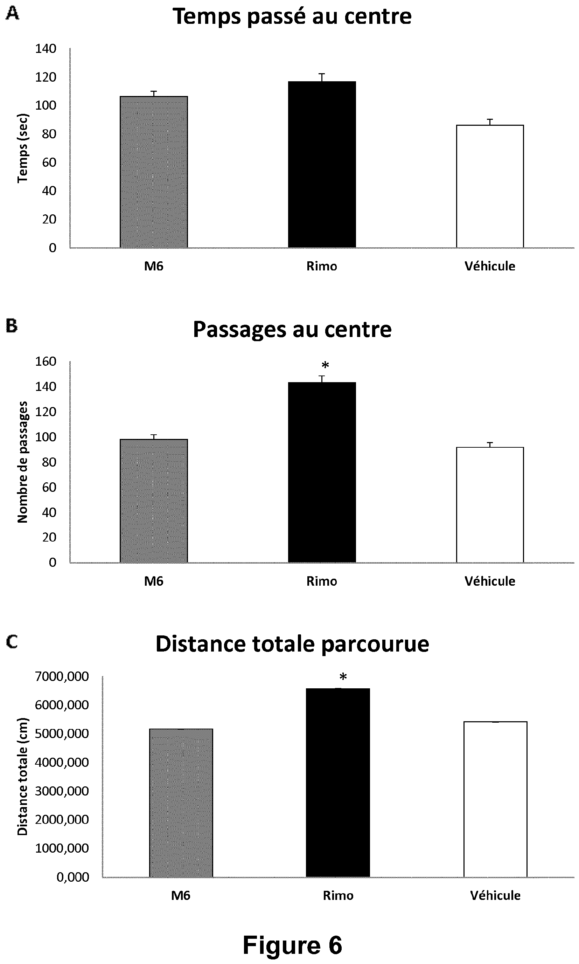

Les résultats du test indiquent tout d'abord que ni l'administration de SR141716 (i.e. Rimonabant) ni celle de JM-00.266 (i.e. composé IA) n'a d'effet significatif sur le temps passé dans le centre de l'arène (zone la plus anxiogène) suggérant que l'état d'anxiété des souris obèses n'a pas été altéré à la fin des 30 jours de traitement (Figure 6). Par contre, il est à noter que le SR141716 (i.e. Rimonabant) augmente l'activité motrice alors que le composé JM-00.266 (i.e. composé IA) n'a aucun effet sur ce paramètre suggérant que JM-00.266 (i.e. composé IA) n'exerce aucune action centrale.

### 2.2.3. Effets du traitement chronique par le composé JM-00.266 (i.e. composé IA) sur la prise alimentaire, le poids et la composition corporelle.

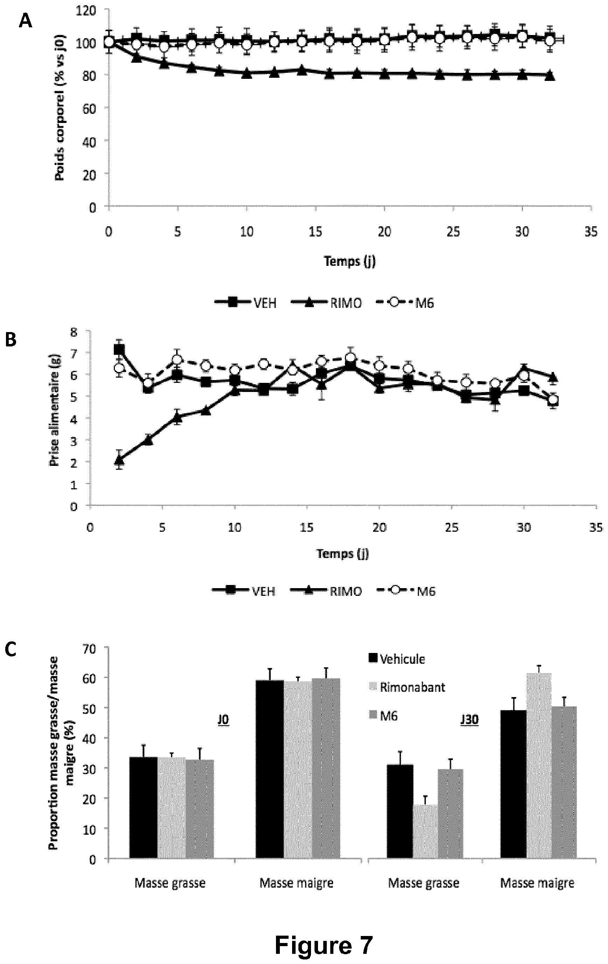

Durant toute la durée du traitement, la prise alimentaire et le poids corporel ont été mesurés tous les deux jours. La Figure 7A montre que la prise alimentaire des animaux traités n'est pas altérée par le traitement JM-00.266 (i.e. composé IA) par rapport à celle des souris témoins ayant reçu le véhicule. Seules les souris ayant reçu le SR141716 (i.e. Rimonabant) voient leur prise alimentaire diminuer transitoirement.

Parallèlement à la prise alimentaire, l'évolution du poids corporel en réponse aux traitements a été surveillée tous les deux jours (Figure 7B). Les résultats montrent que les souris traitées par le SR141716 (i.e. Rimonabant) ont perdu du poids, ce qui est en accord avec la diminution de la prise alimentaire observée. Le poids corporel des animaux traités par JM-00.266 (i.e. composé IA) n'est pas diminué par rapport aux souris témoins.

A la fin du traitement, la composition corporelle (masse grasse, masse maigre) des souris a été analysée par RMN. La Figure 7C montre que seules les souris traités par le SR141716 (i.e. Rimonabant) présentent une masse grasse inférieure et une masse maigre supérieure à celles des souris témoins.

L'effet du SR141716 (i.e. Rimonabant) observé dans cette étude a déjà été décrit dans la littérature et s'explique par l'action centrale du SR141716 sur les CB1R conduisant à une rapide (mais transitoire) diminution de la prise alimentaire suivie d'une perte de masse corporelle (Ravinet Trillou et al., 2003). Le fait que le composé JM-00.266 (i.e. composé IA) n'ait pas d'effet sur la prise alimentaire ni sur le poids, sur l'échelle de temps testée, confirme que l'action du composé est bel et bien limitée aux récepteurs CB1 périphériques.

### 2.2.4. Effets du traitement chronique par le composé JM-00.266 (i.e. composé IA) sur la glycémie et la tolérance au glucose

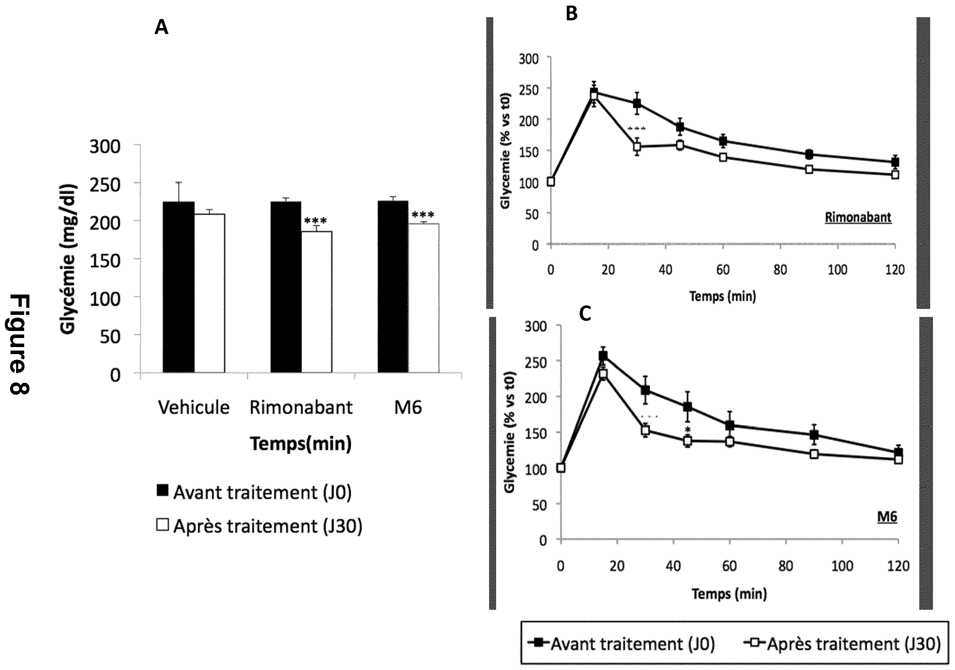

Pour évaluer les effets à long terme du composé JM-00.266 (i.e. composé IA) sur le métabolisme glucidique, des tests de tolérance au glucose (OGTT 2g/kg) ont été réalisés avant et à la fin des traitements. Les résultats indiquent une amélioration significative de la glycémie basale (Figure 8A) et de la tolérance au glucose chez les souris obèses après 30 jours de traitement par le JM-00.266 et le SR141716 (i.e. Rimonabant) (Figure 8B).

### 2.2.5. Effets du traitement chronique par le composé JM-00.266 (i.e. composé IA) sur l'insulinémie et la sensibilité à l'insuline

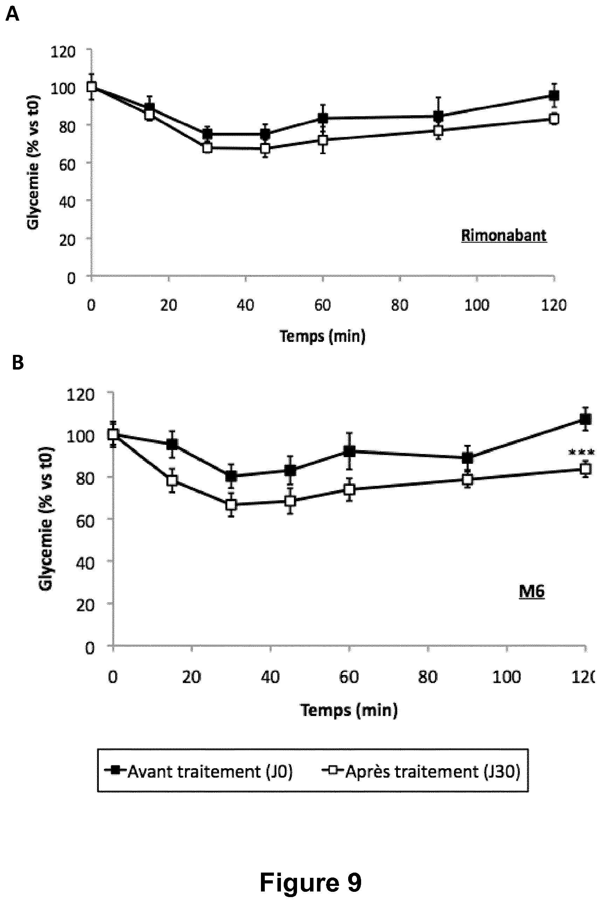

Afin de savoir si l'amélioration du contrôle glycémique induite par l'administration chronique du composé JM-00.266 (i.e. composé IA) peut être associée à une amélioration de la résistance à l'insuline, l'effet du traitement sur la sensibilité à l'insuline en réalisant des ITT a également été mesuré. Ce test démontre que l'insulino-résistance des souris obèses détectée avant le début du traitement est améliorée après l'administration de SR141716 (i.e. Rimonabant) et JM-00.266 (i.e. composé IA) pendant 30 jours (Figure 9). Il est à noter que la diminution de la glycémie induite par l'injection d'insuline est plus marquée chez les souris traitées par JM-00.266 (i.e. composé IA) que chez celles traitées par le SR141716 (i.e. Rimonabant).

### 2.3. Effet in vivo de l'administration à long terme du composé IA (autrement nommé M6 ou JM-00.266) en association avec une diminution de l'apport énergétique sur la souris obèse

### 2.3.1. Effet sur la masse corporelle

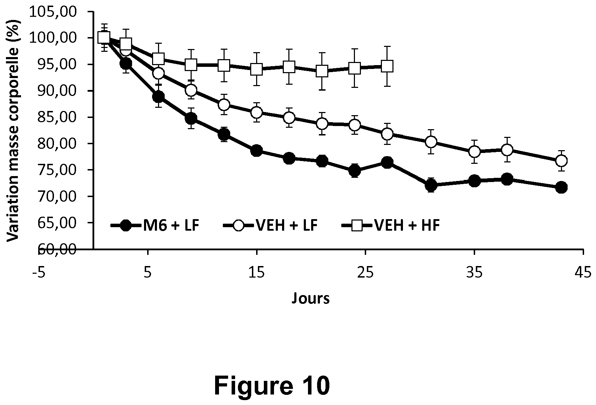

Comme le montrent les résultats présentés sur le graphique en Figure 10 :
- lorsque des souris obèses (46,21±1,18 g) sont maintenues au régime hyperlipidique (30% de lard, 33.5% de glucides) (VEH+HF), leur masse corporelle reste stable ;
- lorsque des souris obèses (46,01±0,84 g) sont nourries avec un régime normolipidique (5% de lipides) (VEH+LF) au lieu d'un régime hyperlipidique à 30% de lard, elles perdent du poids ;
- lorsque des souris obèses (45,83±0,82 g) sont nourries avec un régime normolipidique à 5% de lipides et reçoivent le composé M6 (M6+LF), leur masse diminue significativement plus.

Par conséquent, on a observé que l'administration du composé M6 en complément d'un régime normolipidique renforce la perte de poids induite par une restriction calorique chez des souris préalablement rendues obèses par un régime hyperlipidique.

### 2.3.2. Effet sur la tolérance au glucose

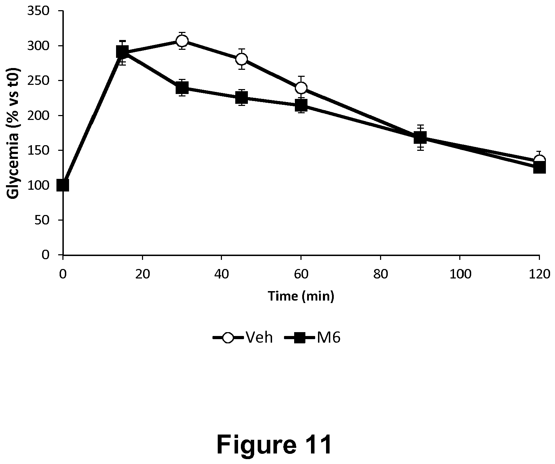

Comme le montrent les résultats présentés sur le graphique en Figure 11 : après 43 jours de traitement avec le composé M6 (JM-00.266) administré par voie orale (10mg/kg) 1 fois par jour ou avec le véhicule, les souris soumises au régime normolipidique (M6 + LF) présentent une meilleure tolérance au glucose par rapport aux témoins respectifs véhicule et régime normolipidique (VEH + LF) et véhicule et régime hyperlipidique (VEH + HF) .

### 2.4. Effet in vivo de l'administration à long terme du composé IA (autrement nommé M6 ou JM-00.266) simultanément à une prise alimentaire chez une souris obèse

### 2.4.1 Effet sur la masse corporelle

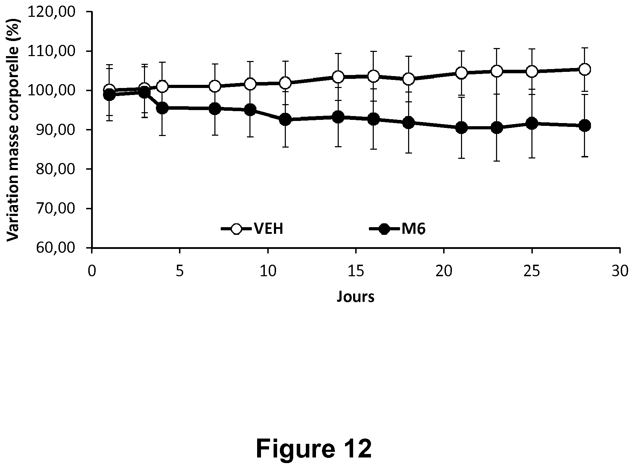

Comme le montrent les résultats présentés sur le graphique en Figure 12 : lorsque des souris obèses (46,81±3,02 g) soumises à un régime hyperlipidique (35% de lard) sont traitées avec le composé M6 à raison d'une administration journalière pendant le repas (M6), elles perdent plus de poids que les souris traitées avec le véhicule (VEH).

Par conséquent, une administration du composé M6 au cours du repas favorise la perte de poids de souris rendues obèses par un régime hyperlipidique et soumises à ce même régime pendant le traitement. Des résultats similaires sont attendus avec une administration du composé M6 juste avant le repas, compte tenu des effets du composé M6 observés sur le contrôle glycémique lorsqu'il est administré en aigu 10 minutes avant une administration de glucose (voir paragraphe 2.1.3 ci-dessus).

### 2.4.2 Effet sur la tolérance au glucose

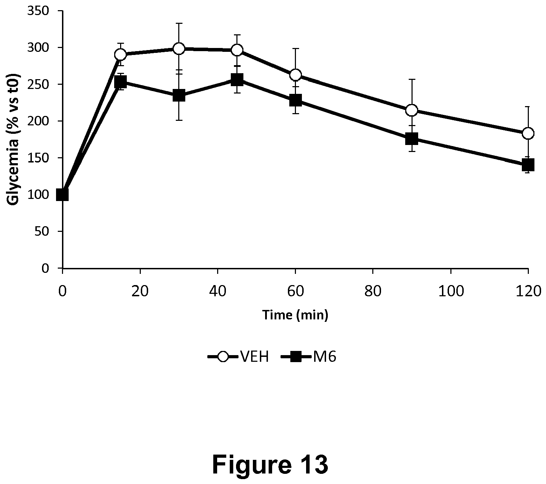

Comme le montrent les résultats présentés sur le graphique en Figure 13 : après 28 jours de traitement par le composé M6, administré en même temps que le repas, les souris présentent une meilleure tolérance au glucose que les souris traitées par une administration de véhicule en même temps que le repas.

### 2.4.3 Effet sur l'expression génique de marqueurs

Le traitement par M6 conduit à une diminution de l'expression de la NAPE-PLD dans les tissus testés suggérant une diminution du tonus endocannabinoide.

L'expression de CB1R et de l'enzyme de dégradation des endocannabinoides (Fatty acid amide hydrolase, FAAH) n'est pas modifiée par le traitement dans aucun des tissus testés. L'expression des CB2R est également plus faible dans les tissus des animaux traités par M6. Les CB2R étant principalement exprimés dans les cellules immunitaires, il est possible que ce résultat traduise une moindre infiltration macrophagique.

La Fatty acid synthase (FAS), Stearoyl- CoA desaturase 1 (SCD-1) et la Glycerol-Phosphate acyl-transferase (GPAT2) sont des enzymes dont les variations d'expression traduisent l'activité lipogénique. Ainsi, la diminution de l'expression de SCD-1 et GPAT dans le foie et FAS dans le tissu adipeux des animaux traités par M6 suggère une moindre synthèse de lipides dans ces tissus.

La Glucose-6-phosphatase (G6P) et les transporteurs du glucose GLUT2 et GLUT4 dans le foie sont utilisés ici comme marqueurs de la néoglucogenèse. Les résultats suggèrent que le traitement M6 pourrait être associé à une moindre production de glucose par le foie.

La diminution de l'expression de la G6P et de GLUT4 dans le tissu adipeux des souris traitées par M6 pourrait refléter une réduction de l'utilisation du glucose engagé dans la voie de la lipogenèse.

F4/80 est un marqueur des macrophages matures. L'expression de F4/80 paraît nettement diminuée dans le foie et les tissus adipeux suggérant une moindre infiltration macrophagique.

Le Tumour necrosis factor-alpha (TNF-a) est une cytokine pro-inflammatoire intervenant dans la régulation de nombreux processus biologiques comme les fonctions immunitaires, la différentiation cellulaire ou le métabolisme énergétique. Il est admis que les macrophages ayant infiltré le tissu adipeux de souris obèses sont responsables d'une élévation de la production de TNF-a. Dans la présente étude, une tendance à la diminution de l'expression de TNF-a dans le tissu adipeux est observée, en association à la diminution de l'expression de F4/80 par M6. Ces résultats semblent en accord avec la diminution de l'expression du CB2R qui est essentiellement localisé sur les cellules immunitaires.

### III. CONCLUSION

L'obésité est associée à une hyperactivation du système endocannabinoïde (SEC) dépendante des récepteurs aux endocannabinoïdes 1 (CB1R). Ainsi, l'inactivation des CB1R constitue une stratégie de traitement pour lutter contre les désordres métaboliques liés à l'obésité. Le SR141716 (Rimonabant), agoniste inverse des CB1R, fut le premier agent anti-obésité commercialisé mais a été néanmoins rapidement retiré du marché en raison d'effets secondaires d'ordre neuropsychiatrique consécutifs au blocage des CB1R centraux. Toutefois, une action ciblée des CB1R périphériques pourrait constituer une solution thérapeutique à part entière pour traiter l'obésité et certaines pathologies qui lui sont associées. En effet, même si la réduction de la prise alimentaire semble être la principale cause de la perte de poids et de l'amélioration des paramètres métaboliques, plusieurs études chez l'animal et chez l'homme indiquent que les CB1R périphériques pourraient également être impliqués dans la régulation du métabolisme glucido-lipidique (Nogueiras et al., 2008 ; Osei-Hyiaman et al., 2008). Par conséquent, il a été proposé que les effets bénéfiques à long terme de l'inactivation du système endocannabinoide sont dus à la fois aux effets centraux sur la prise alimentaire et à des effets périphériques concernant le tissu adipeux, le foie, le muscle squelettique et le pancréas. Le rôle des CB1R périphériques a clairement été démontré par les travaux de Tam et al. en 2010 indiquant que le blocage de ces récepteurs par un antagoniste périphérique diminue le risque cardio-métabolique chez la souris obèse. Deux études récentes sont également en accord avec cette notion. La première suggère qu'une réduction de l'activité du système endocannabinoïde dans le tissu adipeux viscéral est associée à une normalisation du métabolisme adipocytaire favorable à la réversion de la stéatose hépatique observée chez la souris obèse (Jourdan et al., 2010). La seconde démontre par une approche *in vitro* que l'inactivation des CB1R hépatiques conduit à une stimulation de la béta-oxydation des acides gras (Jourdan et al., 2012). L'utilisation de composés agissant comme antagonistes des récepteurs aux endocannabinoïdes de type CB1 (CB1R) présente donc un intérêt certain dans la maîtrise de la régulation de la prise alimentaire et des métabolismes glucidique et lipidique dans des pathologies comme le syndrome métabolique dont l'impact est important en termes de santé publique. Par ailleurs, le développement de composés gardant une activité sur les CB1R périphériques, mais ne franchissant pas la barrière hémato-encéphalique permettrait de contourner les difficultés d'utilisation clinique des molécules de la génération du Rimonabant.

Les présents résultats, basés sur trois approches « *in vitro »*, « *in vivo* court terme » et « *in vivo* long terme », démontrent que, parmi cinq nouvelles molécules testées, seul le composé JM-00.266 présente :
(1) des propriétés agoniste inverse vis-à-vis du récepteurs CB1,
(2) une action limitée à la périphérie et n'est donc pas susceptible d'exercer d'effets secondaires psychotropes délétères comme cela a été le cas avec des composés qui ont antérieurement fait l'objet de tentatives de développement,
(3) des effets bénéfiques sur le métabolisme glucido-lipidique, dans le cadre de l'obésité, en améliorant notamment la tolérance au glucose et la sensibilité à l'insuline, et en diminuant la triglycéridémie, et
(4) une capacité à empêcher l'action inhibitrice de l'anandamide (agoniste naturel du CB1R) sur la motilité gastro-intestinale ce qui démontre un effet antagoniste puissant du composé *in vivo,* ainsi que son utilité dans le traitement de la gastroparésie.

L'effet du composé JM-00.266 *in vivo* sur la masse corporelle et certains paramètres biologiques chez la souris obèse en fonction du mode d'administration a également permis de mettre en évidence les éléments suivants :
- ce composé, administré en complément d'un régime normolipidique, renforce la perte de poids induite par une restriction calorique chez des souris préalablement rendues obèses par un régime hyperlipidique et améliore la tolérance au glucose. Ces résultats permettent d'envisager des traitements combinants ce type de composé à des régimes alimentaires normocaloriques et/ou à des composés connus pour réguler négativement la prise alimentaire ;
- ce composé, administré au cours du repas, favorise la perte de poids des souris rendues obèses par un régime hyperlipidique et soumises à ce même régime pendant le traitement, et améliore la tolérance au glucose. Ces résultats permettent d'envisager des protocoles d'administration du composé d'intérêt de préférence juste avant et/ou pendant le ou les repas;
- l'effet *in vivo* de ce composé sur différents marqueurs de l'activité du système endocannabinoïde, de l'activité lipogénique, et de l'infiltration macrophagique, suggère notamment une diminution du tonus endocannabonoide, une moindre synthèse des lipides dans les tissus, une réduction de l'utilisation du glucose engagé dans la voie de la lipogénèse et une moindre inflitration macrophagique.

Par ailleurs, la structure de ce nouveau composé et les possibilités de pharmacomodulation de celui-ci offrent ainsi la possibilité d'accéder à des molécules diffusant peu ou pas du tout dans le système nerveux central, qui sont représentées par les composés de formule (I) décrits ci-dessus.

Compte tenu des propriétés observées dans le cadre de ces travaux, le composé de formule (I) selon l'invention présente sans conteste un intérêt thérapeutique non seulement vis-à-vis de pathologies liées à l'obésité, mais également pour les pathologies pour lesquelles l'implication des CB1R dans différents tissus périphériques a été mise en évidence. Ainsi, l'inactivation des CB1R périphériques par le composé selon l'invention peut permettre de traiter la résistance à l'insuline (Song et al., 2011 ; Eckardt et al., 2009), le diabète de type II (Matias et al. 2006 ; Jensen, 2006), la stéatose hépatique non alcoolique (Osei-Hyiamann et al., 2005 ; Osei-Hyiaman et al. 2008 ; Jeong et al. 2008), la fibrose hépatique (Teixeira-Clerc et al. 2006), la néphropathie (Jourdan et al., 2012), la fibrose rénale (Lecru et al., 2015), les cardiomyopathies (Montecucco et Di Marzo, 2012 ; Rajesh et al., 2012 ; Slavic et al., 2013 ; Schaich et al., 2014 ; Pacher et Kunos, 2013), la gastroparésie (Izzo et Sharkey, 2010), la croissance osseuse et le développement du cartilage (Tam et al., 2008 ; Wasserman et al., 2015), le développement musculaire (Iannotti et al., 2014), la fertilité, notamment la motilité et viabilité des spermatozoïdes chez l'homme (Amoako et al., 2014) et la nidation de l'ovocyte chez la femme (Wang et al., 2006).

### REFERENCES BIBLIOGRAPHIQUES

Amoako AA et al. (2014). Fertil. Steril.; 102:1260-1267.
Beaumont H, Jensen J et al. (2009). Br J Pharmacol ; 156(1):153-62.
Blüher M, Engeli et al. (2006). Diabetes;55(11):3053-60.
Buckley NE, Hansson S et al. (1998). Neuroscience ; 82(4) :1131-1149.
Cota D, Marsicano G et al. (2003). J clin Invest ; 112(3) :423-431.
Côté M, Matias I, et al. (2007). Int J Obes (Lond); 31(4):692-9.
Das SK, Paria BC et al. (1995). Proc Natl Acad Sci USA ; 92(10) :4332-4336.
Despres JP et Lemieux I (2006). Nature ; 444(7121):881-887.
Di Carlo G et Izzo AA (2003). Expert Opin Investig Drugs ; 12(1) :39-49.
Di Marzo V et Matias I (2005). Nat Neurosci ; 8(5):585-9.
Di Marzo V (2008). Nature Reviews Drug Discovery; 7:438-455.
Di Marzo V et al. (2008). British Journal of Pharmacology ; 153:1272-1280.
Eckardt K et al. (2009). Diabetologia ; 52:664-674.
Gye MC, Kim C et al. (2001). Arch Androl ; 46(1) : 51-55.
Hoehe MR, Carnazzo L et al. (2001). New biol ; 3(9): 880-885.
Iannotti FA et al. (2014). Proc. Natl. Acad. Sci. U.S.A ; 117:2472-2481.
Izzo AA, Mascolo N et al. (1999). Arch Pharmacol. ; 360(2):221-3.
Izzo AA, Fezza F et al. (2001). Br J Pharmaco l; 134(3):563-70.
Izzo AA et Sharkey KA (2010). Pharmacol Ther ; 126(1):21-38.
Jensen M. (2006). The American Journal of Medicine (2007); Vol 120 (9A), S25-S32.
Jeong WI et al. (2008). Cell Metab. ; 7 :227-235.
Jourdan T. et al. (2010). Diabetes ; 59:926-934.
Jourdan T. et al. (2012). Hepatology ; 55:790-799.
Jourdan T, Szanda G et al. (2014). Proc Natl Acad Sci USA ; 111(50):E5420-E5428.
Lehmann A, Blackshaw LA et al. (2002). Gastroenterology ; 123(4):1129-34.
Lecru L. et al. (2015). Kidney Int.; 88(1):72-84
Liu J, Batkai S et al. (2003). J Biol Chem ; 278(45) :45034-45039.
Liu J. et al. (2012). Gastroenterolog ; 142(5):1218-1228.
Matias I et al. (2006). J. Clin. Endocrinol. Metab ; 91:3171-3180.
Maccarrone M, Bab I et al. (2015). Trends Pharmacol Sci. ;36(5):277-296.
Massa F, Marsicano G et al. (2004). J Clin Invest ; 113(8):1202-9.
Nogueiras R et al.(2008). Diabetes ; 57, 2977-2991.
Osei-Hyiaman D et al. (2005). J. Clin. Invest ; 115(5): 1298-1305.
Osei-Hyiaman D et al. (2008). J. Clin. Invest ; 118:3160-3169.
Pacher P et Kunos G (2013). FEBSJ ; 280(9):1918-43.
Pertwee RG (2001). Prog. Neurobiol ; 63(5):569-611.
Rajesh M et al. (2012). Diabetes ; 61:716-727.
Rinaldi-Carmona M et al. (1996). J Pharmacol Exp Ther ; 278(2):871-8.
Ravinet Trillou C et al. (2003). Am J Physiol Regul Integr Comp Physiol ; 284(2): R345-R353.
Schaich CL et al. (2014). Physiol. Rep; 2(8):e12108.
Tam J et al. (2008). FASEB J. ; 22:285-294.
Tam J, Vemuri VK et al. (2010). J Clin Invest ; 120(8):2953-66.
Tam J, Cinar R et al. (2012). Cell Metab ; 16(2):167-79.
Teixeira-Clerc F et al. (2006). Nature Medicine ; 12(6) :671-676.
Slavic S. et al. (2013). J. Mol. Med. ; 91:811-823.
Song D. et al. (2011). Diabetologia ; 54:1181-1189.
Wang H. et al. (2006). Endocr. Rev. 27:427-448

## Revendications

1. Composé de formule (I) suivante : 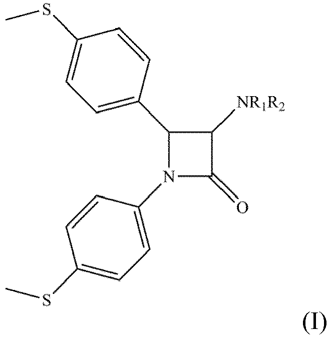 dans laquelle :
• R₁ et R₂, identiques ou différents, représentent un atome d'hydrogène ou un groupe COR₃, SO₂R₄ ou CONR₅R₆; ou forment ensemble avec l'atome d'azote qui les porte un hétérocycle à 5 ou 6 chainons comprenant au moins un hétéroatome supplémentaire, groupe C=O ou groupe aryle;
• R₃, R₄, R₅ et R₆ représentent, indépendamment les uns des autres, un atome d'hydrogène, ou un groupe aryle, ledit groupe étant optionnellement substitué par un ou plusieurs groupe(s) choisi(s) parmi un atome d'halogène, OR₇, NR₈R₉, SR₁₀, S(O)R₁₁, SO₂R₁₂, SO₂NR₁₃R₁₄, OCOR₁₅, NR₁₆COR₁₇, NR₁₈C(O)OR₁₉, CO₂R₂₀, CONR₂₁R₂₂, OCO₂R₂₃, OCONR₂₄R₂₅, COR₂₆, nitro (NO₂), cyano (CN), oxo (=O) et CF₃; et
• R₇ à R₂₆ représentent, indépendamment les uns des autres, un atome d'hydrogène ou un groupe (C₁-C₆)alkyle, aryle ou aryl-(C₁-C₆)alkyle,
ou un sel et/ou un solvate pharmaceutiquement acceptable de celui-ci.

2. Composé selon la revendication 1, **caractérisé en ce que** R₁ et R₂ forment ensemble avec l'atome d'azote qui les porte un hétérocycle de formules (II) ou (III) suivantes : 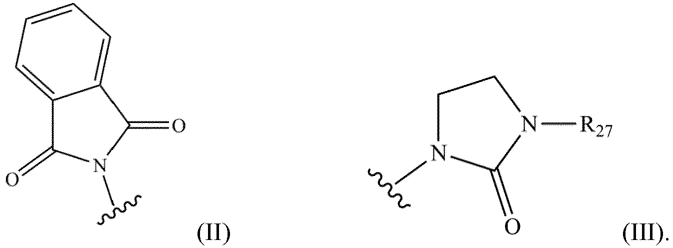 dans laquelle R₂₇ représente un atome d'hydrogène ou un groupe COR₃ ou SO₂R₄, R₃ et R₄ étant tels que définis dans la revendication 1, en particulier R₂₇ représente un atome d'hydrogène.

3. Composé selon la revendication 1, **caractérisé en ce que** :
• R₁ et R₂, identiques ou différents, représentent un atome d'hydrogène ou un groupe COR₃, SO₂R₄ ou CONR₅R₆, préférablement R₁ est un atome d'hydrogène et R₂ représente un groupe COR₃, SO₂R₄ ou CONR₅R₆,
• R₃, R₄ et R₅ représentent, indépendamment les uns des autres, un groupe aryle, de préférence un phényle, optionnellement substitué par un groupe choisi parmi un atome halogène, de préférence Cl ou F, CF₃ et SO₂R₁₂, avec R₁₂ représentant un groupe (C₁-C₆)alkyle, de préférence un méthyle ; et
• R₆ représente un atome d'hydrogène,
ledit composé étant avantageusement choisi parmi les composés suivants :
| | |
|---|---|
| Composé (IA) | Composé (IB) |
| 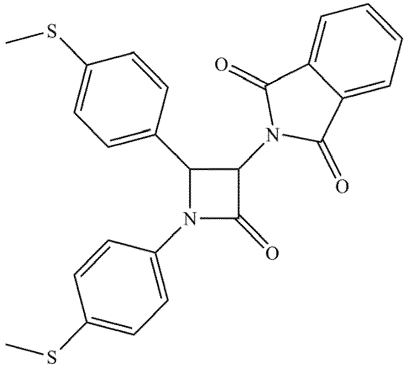 | 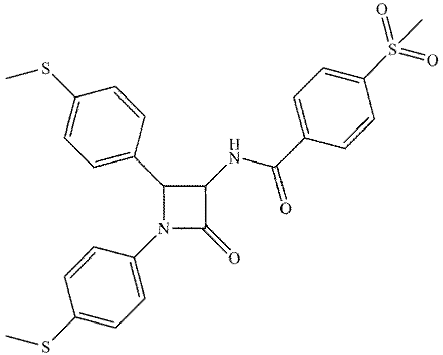 |
| Composé (IC) | Composé (ID) |
| 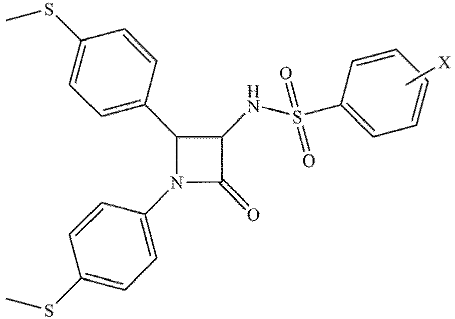 | 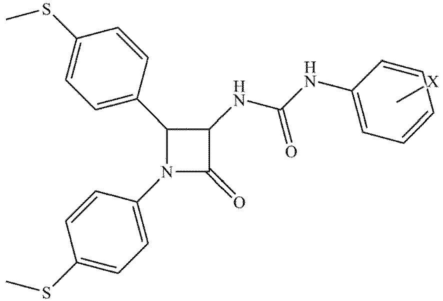 |
| Composé (IE) | Composé (IF) |
| 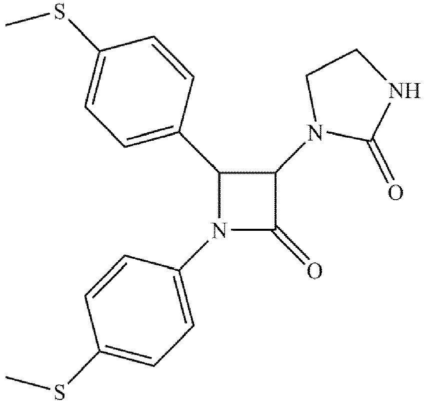 | 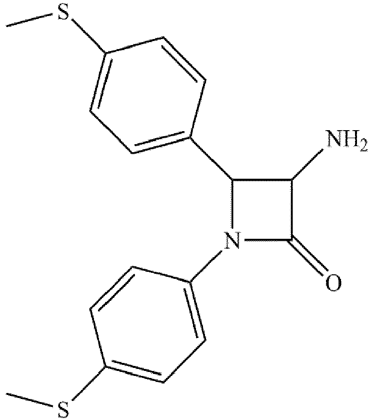 |
ou un sel et/ou un solvate pharmaceutiquement acceptable de ceux-ci, dans lesquels X représente un atome d'hydrogène, un halogène ou CF₃.

4. Procédé de préparation d'un composé de formule (I) tel que défini selon l'une quelconque des revendications 1 à 3, comprenant les étapes suivantes :
(i) condensation du 4-méthylthiobenzaldéhyde avec la 4-méthylthio aniline pour obtenir le composé de formule (IV) suivante : 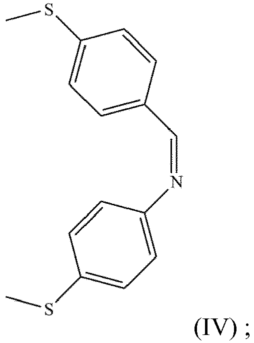
(ii) cycloaddition de Staudinger entre le composé de formule (IV) obtenu et un cétène de formule (V) suivante : 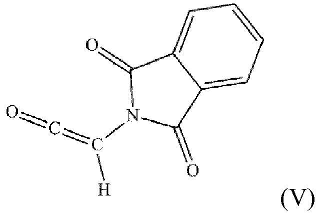 pour obtenir le composé de formule (IA) suivante : 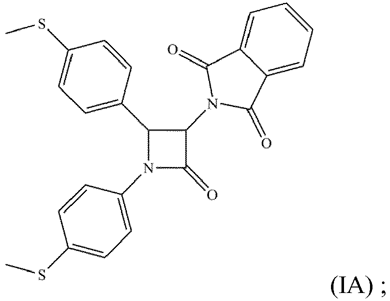
(iii) optionnellement, déprotection de la fonction amine phtaloylée du composé de formule (IA), de préférence par action de la méthylhydrazine, pour obtenir le composé de formule (IF) suivante : 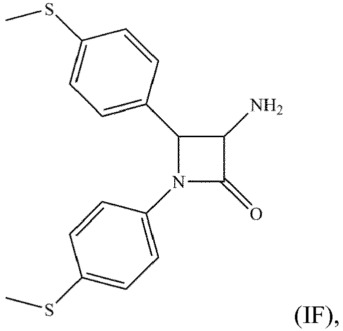 puis, optionnellement, couplage du composé de formule (IF) ainsi obtenu avec un composé de formule R₁-X et/ou R₂-X', dans laquelle R₁-X et R₂-X' sont des formes activées, telles que chlorures d'acyles, chlorures de sulfonyles et isocyanates d'aryles, des groupements R₁ et R₂ tels que définis dans la revendication 1 ; et
(iv) récupération du composé obtenu à l'étape (ii) ou à l'étape (iii).

5. Utilisation *in vitro* d'au moins un composé de formule (I) tel que défini selon l'une quelconque des revendications 1 à 3, en tant qu'agoniste inverse des récepteurs endocannabinoïdes CB1 périphériques.

6. Composition pharmaceutique comprenant en tant que principe actif au moins un composé de formule (I) tel que défini selon l'une quelconque des revendications 1 à 3, et au moins un excipient pharmaceutiquement acceptable.

7. Composition non thérapeutique, comprenant au moins un composé de formule (I) tel que défini selon l'une quelconque des revendications 1 à 3, et au moins un excipient acceptable.

8. Composé tel que défini selon l'une quelconque des revendications 1 à 3, ou composition pharmaceutique telle que définie selon la revendication 6, pour son utilisation comme médicament.

9. Composé tel que défini selon l'une quelconque des revendications 1 à 3, ou composition pharmaceutique telle que définie selon la revendication 6, pour son utilisation dans la prévention ou le traitement de pathologies associées à une hyperactivité du système endocannabinoïde, les dites pathologies étant sélectionnées parmi l'obésité et les troubles métaboliques qui y sont associés, la résistance à l'insuline, le diabète et les complications associées, la stéatose hépatique, la fibrose hépatique, la cirrhose, la fibrose rénale, la néphropathie, les cardiomyopathies, la gastroparésie, la perte d'os et/ou de cartilage, la perte de muscle, et les troubles de la fertilité.

10. Composé ou composition pharmaceutique pour son utilisation selon la revendication 9, **caractérisé en ce que** ledit composé ou ladite composition est administrée à un sujet soumis à un régime alimentaire normocalorique équilibré.

11. Composé ou composition pharmaceutique pour son utilisation selon la revendication 9 ou 10, **caractérisé en ce que** ledit composé ou ladite composition est administré(e) audit sujet avant et/ou pendant le ou les repas du sujet.

12. Composé de formule (I) tel que défini selon l'une quelconque des revendications 1 à 3, et agent thérapeutique destiné à prévenir ou traiter une pathologie associée à une hyperactivité du système endocannabinoïde, en tant que préparation combinée pour une administration simultanée, séparée, ou étalée dans le temps.

13. Méthode non thérapeutique pour promouvoir et/ou accélérer la prise de poids ou pour ralentir et/ou atténuer la prise du poids chez un sujet, comprenant l'administration d'une quantité efficace d'un composé de formule (I) tel que défini selon l'une quelconque des revendications 1 à 3, ou d'une composition telle que définie selon la revendication 7, ledit sujet étant préférablement soumis à un régime alimentaire normocalorique équilibré.

14. Méthode non thérapeutique selon la revendication 15, dans laquelle ledit composé ou ladite composition est administré(e) audit sujet avant et/ou pendant le ou les repas du sujet.

## Patentansprüche

1. Verbindung, der folgenden Formel (I): 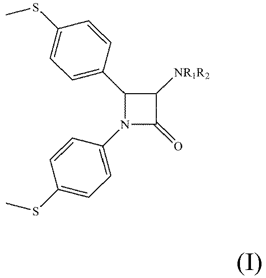 wobei:
• R₁ und R₂, identisch oder unterschiedlich, ein Wasserstoffatom oder eine COR₃, SO₂R₄ oder CONR₅R₆ Gruppe repräsentieren; oder gemeinsam mit dem Stickstoffatom, das sie trägt, einen Heterozyklus mit 5 oder 6 Ketten bilden, der mindesten ein zusätzliches Heteroatom, eine C=O-Gruppe oder Arylgruppe umfasst;
• R₃, R₄, R₅ und R₆ unabhängig voneinander ein Wasserstoffatom, oder eine Arylgruppe repräsentieren, wobei die Gruppe optional durch eine oder mehrere Gruppe(n) substituiert ist, ausgewählt aus einem Halogenatom OR₇, NR₈R₉, SR₁₀, S(O)R₁₁, SO₂R₁₂, SO₂NR₁₃R₁₄, OCOR₁₅, NR₁₆COR₁₇, NR₁₈C(O)OR₁₉, CO₂R₂₀, CONR₂₁R₂₂, OCO₂R₂₃, OCONR₂₄R₂₅, COR₂₆, Nitro (NO₂), Cyano (CN), Oxo (=O) und CF₃; und
• R₇ bis R₂₆ unabhängig voneinander ein Wasserstoffatom oder eine (Ci-C₆)Alkyl-, Aryl- oder Aryl-(C₁-C₆)Alkyl- Gruppe repräsentieren,
oder ein Salz und/ oder ein pharmazeutisch annehmbares Solvat desselben.

2. Verbindung nach Anspruch 1, **dadurch gekennzeichnet, dass** R₁ und R₂ gemeinsam mit dem Stickstoffatom, das sie trägt, einen Heterozyklus der folgenden Formeln (II) oder (III) bilden: 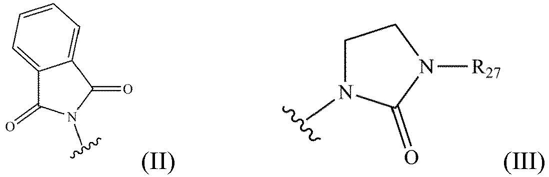 wobei R₂₇ ein Wasserstoffatom oder eine COR₃ oder SO₂R₄ Gruppe repräsentiert, R₃ und R₄ nach Anspruch 1 sind, R₂₇ im Speziellen ein Wasserstoffatom repräsentiert.

3. Verbindung nach Anspruch 1, **dadurch gekennzeichnet, dass**:
• R₁ und R₂, identisch oder unterschiedlich, ein Wasserstoffatom oder eine COR₃, SO₂R₄ oder CONR₅R₅ Gruppe repräsentieren, R₁ vorzugsweise ein Wasserstoffatom ist, und R₂ eine COR₃, SO₂R₄ oder CONR₅R₆ Gruppe repräsentiert,
• R₃, R₄ und R₅ unabhängig voneinander, eine Aryl-Gruppe, vorzugsweise ein Phenyl repräsentieren, optional durch eine Gruppe substituiert, die aus einem Halogenatom, vorzugsweise Cl oder F, CF₃ und SO₂R₁₂ ausgewählt wird, mit R₁₂, das eine (C₁-C₆)Alkyl-, vorzugsweise Methyl-Gruppe repräsentiert; und
• R₆ ein Wasserstoffatom repräsentiert,
wobei die Verbindung vorteilshalber aus den folgenden Verbindungen ausgewählt wird:
| | |
|---|---|
| Verbindung (IA) | Verbindung (IB) |
| 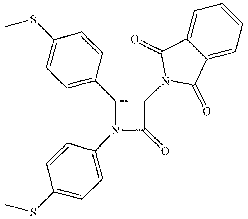 | 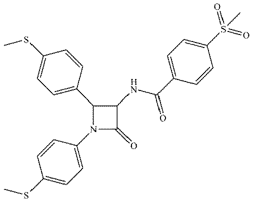 |
| Verbindung (IC) | Verbindung (ID) |
| 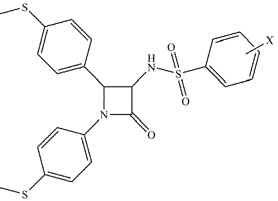 | 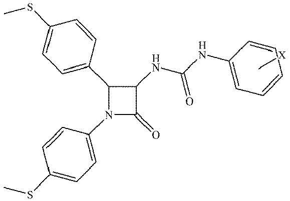 |
| Verbindung (IE) | Verbindung (IF) |
| 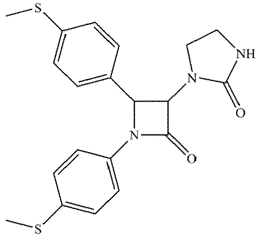 | 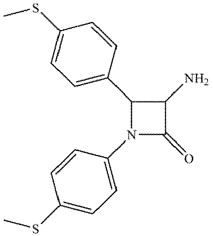 |
oder ein Salz und/ oder ein pharmazeutisch annehmbares Solvat desselben,
in denen X ein Wasserstoffatom, ein Halogen oder CF₃ repräsentiert.

4. Verfahren zum Herstellen einer Verbindung der Formel (I) nach einem der Ansprüche 1 bis 3, die folgenden Schritte umfassend:
(i) Kondensieren des 4-Methylthiobenzaldehyds mit dem 4-Methylthioanilin, um die Verbindung der folgenden Formel (IV) zu erhalten: 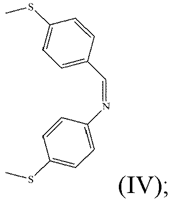
(ii) Staudinger-Cycloaddition zwischen der erhaltenen Verbindung der Formel (IV) und einem Keten der folgenden Formel (V): 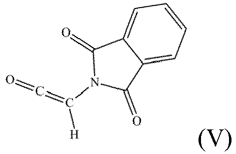 um die Verbindung der folgenden Formel (IA) zu erhalten: 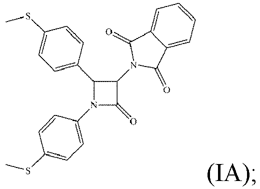
(iii) optional Entschützen der Phthaloyl-Amino-Funktion der Verbindung der Formel (IA), vorzugsweise durch die Wirkung von Methylhydrazin, um die Verbindung der folgenden Formel (IF) zu erhalten: 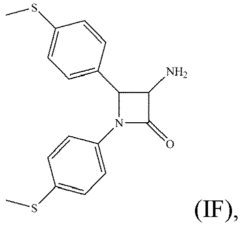 danach, optional Koppeln der so erhaltenen Verbindung der Formel (IF) mit einer Verbindung der Formel R₁-X und/oder R₂-X', wobei R₁-X und R₂-X' aktivierte Formen, wie Acylchloride, Sulfonylchloride und Arylisocyanate, der R₁ und R₂ Gruppierungen nach Anspruch 1 sind; und
(iv) Wiedergewinnen der im Schritt (ii) oder dem Schritt (iii) erhaltenen Verbindung.

5. Verwendung *in vitro* mindestens einer Verbindung der Formel (I) nach einem der Ansprüche 1 bis 3, als inversen Agonisten der peripheren Endocannabinoid CB-1-Rezeptoren.

6. Pharmazeutische Zusammensetzung, als Wirkstoff mindestens eine Verbindung der Formel (I) nach einem der Ansprüche 1 bis 3, und mindestens einen pharmazeutisch annehmbaren Hilfsstoff umfassend.

7. Nicht therapeutische Zusammensetzung, mindestens eine Verbindung der Formel (I) nach einem der Ansprüche 1 bis 3, und mindestens einen annehmbaren Hilfsstoff umfassend.

8. Verbindung nach einem der Ansprüche 1 bis 3, oder pharmazeutische Zusammensetzung nach Anspruch 6, zu deren Verwendung als Arzneimittel.

9. Verbindung nach einem der Ansprüche 1 bis 3, oder pharmazeutische Zusammensetzung nach Anspruch 6, zu deren Verwendung bei der Vorbeugung oder Behandlung von Pathologien im Zusammenhang mit einer Überfunktion des Endocannabinoid-Systems, wobei die Pathologien aus Fettleibigkeit und damit verbundenen Stoffwechselstörungen, Insulinresistenz, Diabetes und den damit verbundenen Komplikationen, Fettleber, Leberfibrose, Zirrhose, Nierenfibrose, Nephropathie, Kardiomyopathien, Magenlähmung, Knochen- und/ oder Knorpelschwund, Muskelschwund, und Fertilitätsstörungen ausgewählt sind.

10. Verbindung oder pharmazeutische Zusammensetzung für deren Verwendung nach Anspruch 9, **dadurch gekennzeichnet, dass** die Verbindung oder die Zusammensetzung einem Patienten verabreicht wird, der einem ausgewogenen normkalorischen Ernährungsplan unterliegt.

11. Verbindung oder pharmazeutische Zusammensetzung für deren Verwendung nach Anspruch 9 oder 10, **dadurch gekennzeichnet, dass** die Verbindung oder die Zusammenstellung dem Patienten vor und/ oder während der Mahlzeit(en) des Patienten verabreicht wird.

12. Verbindung der Formel (I) nach einem der Ansprüche 1 bis 3, und Therapeutikum, das dazu bestimmt ist, einer Pathologie im Zusammenhang mit einer Überfunktion des Endocannabinoid-Systems vorzubeugen oder diese zu behandeln, als Kombinationszubereitung für eine gleichzeitige, getrennte oder zeitversetzte Verabreichung.

13. Nicht therapeutisches Verfahren zum Fördern und/ oder Beschleunigen von Gewichtszunahme oder zum Verzögern und/ oder Abschwächen von Gewichtszunahme bei einem Patienten, umfassend die Verabreichung einer wirksamen Menge einer Verbindung der Formel (I) nach einem der Ansprüche 1 bis 3, oder einer Zusammensetzung nach Anspruch 7, wobei der Patient vorzugsweise einem ausgewogenen normkalorischen Ernährungsplan unterliegt.

14. Nicht therapeutisches Verfahren nach Anspruch 13, wobei die Verbindung oder die Zusammensetzung dem Patienten vor und/ oder während der Mahlzeit(en) des Patienten verabreicht wird.

## Claims

1. Compound of the following formula (I): 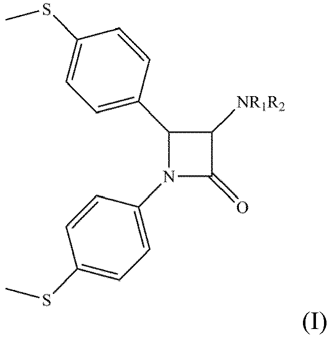 wherein:
• R₁ and R₂, identical or different, represent a hydrogen atom or a group COR₃, SO₂R₄ or CONR₅R₆; or form together with the nitrogen atom bearing same a 5- or 6-membered heterocycle comprising at least one additional heteroatom, C=O group or aryl group;
• R₃, R₄, R₅ and R₆ represent, independently of one another, a hydrogen atom, or an aryl group, said group being optionally substituted by one or a plurality of group(s) chosen from a halogen atom, OR₇, NR₈R₉, SR₁₀,S(O)R₁₁, SO₂R₁₂, SO₂NR₁₃R₁₄, OCOR₁₅, NR₁₆COR₁₇, NR₁₈C(O)OR₁₉, CO₂R₂₀,CONR₂₁R₂₂, OCO₂R₂₃, OCONR₂₄R₂₅, COR₂₆, nitro (NO₂), cyano (CN), oxo (=O) and CF₃; and
• R₇ to R₂₆ represent, independently of one another, a hydrogen atom or a (Ci-C₆)alkyl, aryl or aryl-(C₁-C₆)alkyl group,
or a salt and/or a pharmaceutically acceptable solvate thereof.

2. Compound according to claim 1, **characterised in that** R₁ and R₂ form together with the nitrogen atom bearing same a heterocycle of the following formulas (II) or (III): 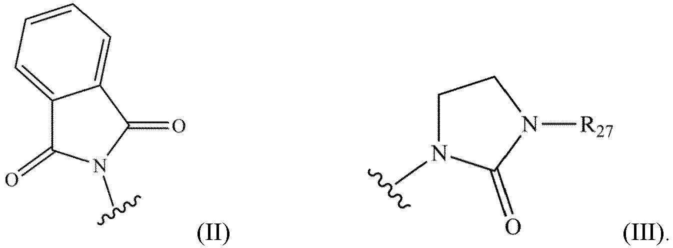 wherein R₂₇ represents a hydrogen atom or a group COR₃ or SO₂R₄, R₃ and R₄ being as defined in claim 1, in particular R₂₇ represents a hydrogen atom.

3. Compound according to claim 1, **characterised in that**:
• R₁ and R₂, identical or different, represent a hydrogen atom or a group COR₃, SO₂R₄ or CONR₅R₆, preferably R₁ is a hydrogen atom and R₂ represents a group COR₃, SO₂R₄ or CONR₅R₆,
• R₃, R₄ and R₅ represent, independently of one another, an aryl group, preferably a phenyl, optionally substituted by a group chosen from a halogen atom, preferably Cl or F, CF₃ and SO₂R₁₂, with R₁₂ representing a (C₁-C₆)alkyl group, preferably a methyl; and
• R₆ represents a hydrogen atom,
said compound being advantageously chosen from the following compounds:
| | |
|---|---|
| Compound (IA) | Compound (IB) |
| 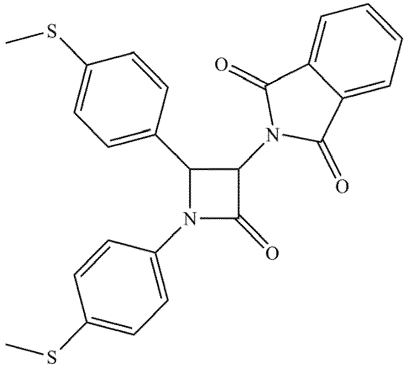 | 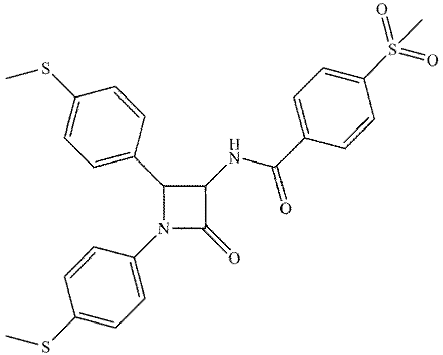 |
| Compound (IC) | Compound (ID) |
| 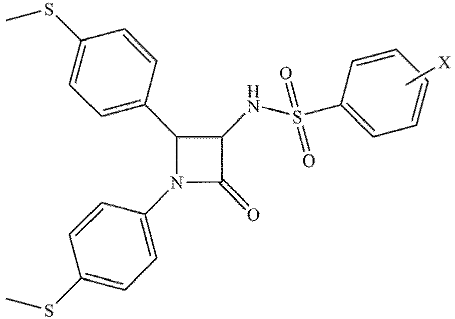 | 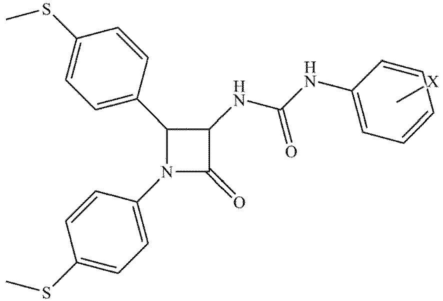 |
| Compound (IE) | Compound (IF) |
| 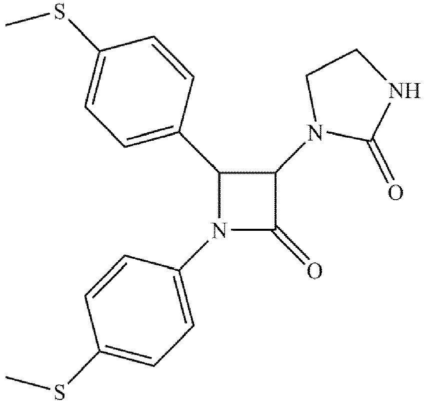 | 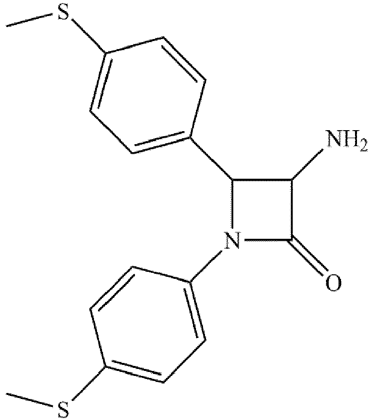 |
or a salt and/or a pharmaceutically acceptable solvate thereof, wherein X represents a hydrogen atom, a halogen or CF₃.

4. Method for preparing a compound of formula (I) as defined according to any one of claims 1 to 3, comprising the following steps:
(i) condensation of 4-methylthiobenzaldehyde with 4-methylthioaniline to obtain the compound of the following formula (IV): 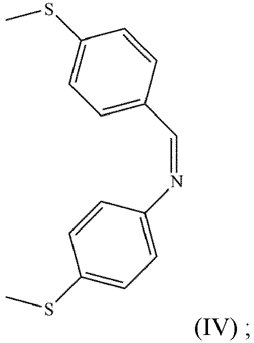
(ii) Staudinger cycloaddition between the compound of formula (IV) obtained and a ketene of the following formula (V): 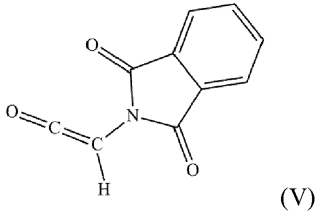 to obtain the compound of the following formula (IA): 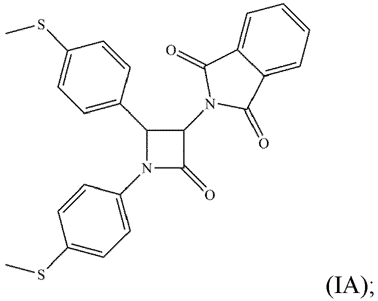
(iii) optionally, deprotection of the phthaloylated amine function of formula (IA), preferably by methylhydrazine action, to obtain the compound of the following formula (IF): 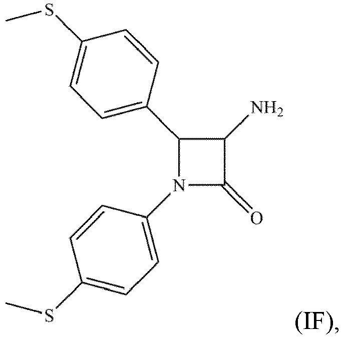 then, optionally, coupling of the compound of formula (IF) obtained with a compound of formula R₁-X and/or R₂-X', wherein R₁-X and R₂-X' are activated forms, such as acyl chlorides, sulphonyl chlorides and aryl isocyanates, of the groups R₁ and R₂ as defined in claim 1; and
(iv) recovery of the compound obtained in step (ii) or in step (iii).

5. In-vitro use of at least one compound of formula (I) as defined according to any one of claims 1 to 3, as an inverse agonist of peripheral CB1 endocannabinoid receptors.

6. Pharmaceutical composition comprising as an active substance at least one compound of formula (I) as defined according to any one of claims 1 to 3, and at least one pharmaceutically acceptable excipient.

7. Non-therapeutic composition, comprising at least one compound of formula (I) as defined according to any one of claims 1 to 3, and at least one acceptable excipient.

8. Compound as defined according to any of claims 1 to 3, or pharmaceutical composition as defined according to claim 6, for use thereof as a medicinal product.

9. Compound as defined according to any one of claims 1 to 3, or pharmaceutical composition as defined according to claim 6, for use thereof in the prevention or treatment of pathologies associated with endocannabinoid system hyperactivity, said pathologies being selected from obesity and the metabolic disorders associated therewith, insulin resistance, diabetes and associated complications, hepatic steatosis, hepatic fibrosis, cirrhosis, renal fibrosis, nephropathy, cardiomyopathies, gastroparesis, bone and/or cartilage loss, muscle loss, and fertility disorders.

10. Compound or pharmaceutical composition for use thereof according to claim 9, **characterised in that** said compound or said composition is administered to a subject on a balanced normocaloric diet.

11. Compound or pharmaceutical composition for use thereof according to claim 9 or 10, **characterised in that** said compound or said composition is administered to said subject before and/or during the subject's meal(s).

12. Compound of formula (I) as defined according to any one of claims 1 to 3, and therapeutic agent intended to prevent or treat a pathology agent associated with endocannabinoid system hyperactivity, as a combined preparation for simultaneous, separate, or time-staggered administration.

13. Non-therapeutic method for promoting and/or accelerating weight gain or for slowing down and/or reducing weight gain in a subject, comprising the administration of an effective quantity of a compound of formula (I) as defined according to any one of claims 1 to 3, or of a composition as defined according to claim 7, said subject being preferably on a balanced normocaloric diet.

14. Non-therapeutic method according to claim 13, wherein said compound or said composition is administered to said subject before and/or during the subject's meal(s).


### SEQUENCE LISTING

<110> UNIVERSITE DE NANTES
   UNIVERSITE DE BOURGOGNE
   INSTITUT NATIONAL DE LA SANTE ET DE LA RECHERCHE MEDICALE (INSERM)
<120> 1,4-DI-(4-METHYLTHIOPHENYL)-3-PHTALOYLAZETIDINE-2-ONE ET SES DERIVES
<130> B371132D34726
<150> FR1559067
   <151> 2015-09-25
<160> 26
<170> PatentIn version 3.5
<210> 1
   <211> 20
   <212> DNA
   <213> artificial
<220>
   <223> TATABox Binding Protein amorce sens
<400> 1
   acggcacagg acttactcca 20
<210> 2
   <211> 22
   <212> DNA
   <213> artificial
<220>
   <223> TATABox Binding Protein amorce antisens
<400> 2
   gctgtctttg ttgctcttcc aa 22
<210> 3
   <211> 20
   <212> DNA
   <213> artificial
<220>
   <223> CB1R amorce sens
<400> 3
   ccgcaaagat agtcccaatg 20
<210> 4
   <211> 19
   <212> DNA
   <213> artificial
<220>
   <223> CB1R amorce antisens
<400> 4
   aaccccaccc agtttgaac 19
<210> 5
   <211> 20
   <212> DNA
   <213> artificial
<220>
   <223> CB2R amorce sens
<400> 5
   caaaggagga agtgcttggt 20
<210> 6
   <211> 21
   <212> DNA
   <213> artificial
<220>
   <223> CB2R amorce antisens
<400> 6
   tggagagatc ggcttatgtt g 21
<210> 7
   <211> 20
   <212> DNA
   <213> artificial
<220>
   <223> F4/80 amorce sens
<400> 7
   tgacaaccag acggcttgtg 20
<210> 8
   <211> 22
   <212> DNA
   <213> artificial
<220>
   <223> F4/80 amorce antisens
<400> 8
   gcaggcgagg aaaagatagt gt 22
<210> 9
   <211> 19
   <212> DNA
   <213> artificial
<220>
   <223> FAAH amorce sens
<400> 9
   ggaccttgct cccctttct 19
<210> 10
   <211> 19
   <212> DNA
   <213> artificial
<220>
   <223> FAAH amorce antisens
<400> 10
   cctgctgggc tgtcacata 19
<210> 11
   <211> 20
   <212> DNA
   <213> artificial
<220>
   <223> FAS amorce sens
<400> 11
   ggctgcagtg aatgaatttg 20
<210> 12
   <211> 20
   <212> DNA
   <213> artificial
<220>
   <223> FAS amorce antisens
<400> 12
   ttcgtacctc cttggcaaac 20
<210> 13
   <211> 20
   <212> DNA
   <213> artificial
<220>
   <223> G6P amorce sens
<400> 13
   tggcctggct tattgtacct 20
<210> 14
   <211> 20
   <212> DNA
   <213> artificial
<220>
   <223> G6P amorce antisens
<400> 14
   gtgctaagag gaagacccga 20
<210> 15
   <211> 19
   <212> DNA
   <213> artificial
<220>
   <223> GLUT2 amorce sens
<400> 15
   ctcttcacca actggccct 19
<210> 16
   <211> 21
   <212> DNA
   <213> artificial
<220>
   <223> GLUT2 amorce antisens
<400> 16
   cagcagatag gccaagtagg a 21
<210> 17
   <211> 21
   <212> DNA
   <213> artificial
<220>
   <223> GLUT4 amorce sens
<400> 17
   gatgccgtcg ggtttccagc a 21
<210> 18
   <211> 21
   <212> DNA
   <213> artificial
<220>
   <223> GLUT4 amorce antisens
<400> 18
   tgttccagtc actcgctgcc g 21
<210> 19
   <211> 22
   <212> DNA
   <213> artificial
<220>
   <223> DGAT2 amorce sens
<400> 19
   agccctccaa gacatcttct ct 22
<210> 20
   <211> 19
   <212> DNA
   <213> artificial
<220>
   <223> DGAT2 amorce antisens
<400> 20
   tgcagctgtt tttccacct 19
<210> 21
   <211> 20
   <212> DNA
   <213> artificial
<220>
   <223> NAPE-PLD amorce sens
<400> 21
   ctcgatatct gcgtggaaca 20
<210> 22
   <211> 20
   <212> DNA
   <213> artificial
<220>
   <223> NAPE-PLD amorce antisens
<400> 22
   ctgaattctg gcgctttctc 20
<210> 23
   <211> 20
   <212> DNA
   <213> artificial
<220>
   <223> SCD1 amorce sens
<400> 23
   ccggagaccc cttagatcga 20
<210> 24
   <211> 26
   <212> DNA
   <213> artificial
<220>
   <223> SCD1 amorce antisens
<400> 24
   tagcctgtaa aagatttctg caaacc 26
<210> 25
   <211> 21
   <212> DNA
   <213> artificial
<220>
   <223> TNF-a amorce sens
<400> 25
   cggggtgatc ggtccccaaa g 21
<210> 26
   <211> 21
   <212> DNA
   <213> artificial
<220>
   <223> TNF-a amorce antisens
<400> 26
   tggtttgcta cgacgtgggc t 21